(19)

Europäisches
Patentamt
European
Patent Office
Office européen
des brevets

(11) **EP 3 836 152 A1**

(12) **EUROPEAN PATENT APPLICATION**

(43) Date of publication:
   16.06.2021 Bulletin 2021/24

(51) Int Cl.:
   *G16H 10/40* (2018.01)       *G16H 50/80* (2018.01)
   *G01N 33/543* (2006.01)       *B01L 3/00* (2006.01)

(21) Application number: 20211391.6

(22) Date of filing: 02.12.2020

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**
   Designated Extension States:
   **BA ME**
   **KH MA MD TN**

(30) Priority: 13.12.2019   US 201916714421
             25.06.2020   US 202016912568
             28.10.2020   US 202017083113

(71) Applicant: **Autonomous Medical Devices
   Incorporated**
   **Inglewood, CA 90304 (US)**

(72) Inventors:
   • **Shachar, Josh**
     **Santa Monica, CA California 90405 (US)**
   • **Kornberg, Roger**
     **Atherton, ca California 94207 (US)**

(74) Representative: **Arnold & Siedsma**
   **Bezuidenhoutseweg 57**
   **2594 AC The Hague (NL)**

(54)   **SYSTEM AND METHOD FOR POINT-OF-CARE, RAPID, FIELD-DEPLOYABLE DIAGNOSTIC
       TESTING OF VIRUSES**

(57)   The present invention relates to an automated system communicated to a remote server for diagnostically field testing a sample taken from a subject using an automated portable handheld instrument to determine the presence of viral antigens and/or antibodies thereto, comprising a microfluidic circuit defined in a rotatable disk, a bio-detector operationally positioned in the microfluidic circuit, wherein the microfluidic circuit is configured for performing a bioassay using the bio-detector to generate an electrical signal indicative of a bioassay measurement, one or more lasers, one or more positionable valves in the microfluidic circuit, and a backbone unit for rotating the disk according to a predetermined protocol to perform the bioassay, for controlling and powering the one or more lasers to selectively open one or more positionable valves in the microfluidic disk, for operating the bio-detector to generate the electrical signal indicative of the bioassay measurement; for communicating the bioassay measurement to the remote server, and for associating the performed bioassay and its corresponding bioassay measurement to the subject.

FIG. 21B

EP 3 836 152 A1

**Description**

The invention relates to the field of point-of-care (POC) pathogen and multiplexed pathogen and antibody array detection platforms and methods, such as in CPC C40B 60/12.

COVID-19 testing involves analyzing samples to assess the current or past presence of SARS-CoV-2. The two main branches detect either the presence of the virus or of antibodies produced in response to infection. Tests for viral presence are used to diagnose individual cases and to allow public health authorities to trace and contain outbreaks. Antibody tests instead show whether someone once had the disease. They are less useful for diagnosing current infections because antibodies may not develop for weeks after infection. They are used to assess disease prevalence, which aids the estimation of the infection fatality rate. Individual jurisdictions have adopted varied testing protocols, including whom to test, how often to test, analysis protocols, sample collection and the uses of test results. This variation has likely significantly impacted reported statistics, including case and test numbers, case fatality rates and case demographics.

Test analysis is often performed in automated, high-throughput, medical laboratories by medical laboratory scientists. Alternatively, point-of-care testing can be done in physician's offices, workplaces, institutional settings or transit hubs. Positive viral tests indicate a current infection, while positive antibody tests indicate a prior infection. Other techniques include a chest CT scan, checking for elevated body temperature or checking for low blood oxygen level.

*Detection of the virus*

*Reverse transcription polymerase chain reaction*

Polymerase chain reaction (PCR) is a process that amplifies or replicates a small, well-defined segment of DNA many hundreds of thousands of times, creating enough of it for analysis. Test samples are treated with certain chemicals that allow DNA to be extracted. Reverse transcription converts RNA into DNA. Reverse transcription polymerase chain reaction (RT-PCR) first uses reverse transcription to obtain DNA, followed by PCR to amplify that DNA, creating enough to be analyzed. RT-PCR can thereby detect SARS-CoV-2, which contains only RNA. The RT-PCR process generally requires a few hours.

Real-time PCR (qPCR) provides advantages including automation, higher-throughput, and more reliable instrumentation. It has become the preferred method. The combined technique has been described as real-time RT- PCR or quantitative RT-PCR and is sometimes abbreviated qRT-PCR, rRT- PCR, or RT-qPCR, although sometimes RT-PCR or PCR are used. The Minimum Information for Publication of Quantitative Real-Time PCR Experiments (MIQE) guidelines propose the term RT-qPCR, but not all authors adhere to this.

Samples can be obtained by various methods, including a nasopharyngeal swab, sputum (coughed up material), throat swabs, and deep airway material collected via suction catheter or saliva. It has been remarked that for 2003 SARS, "from a diagnostic point of view, it is important to note that nasal and throat swabs seem less suitable for diagnosis, since these materials contain considerably less viral RNA than sputum, and the virus may escape detection if only these materials are tested." The likelihood of detecting the virus depends on collection method and how much time has passed since infection. Some have found that tests performed with throat swabs are reliable only in the first week. Thereafter the virus may abandon the throat and multiply in the lungs. In the second week, sputum or deep airways collection is preferred. Collecting saliva may be as effective as nasal and throat swabs, although this is not certain. Sampling saliva may reduce the risk for health care professionals by eliminating close physical interaction. It is also more comfortable for the patient. Quarantined people can collect their own samples. A saliva test's diagnostic value depends on sample site (deep throat, oral cavity, or salivary glands). One study found that saliva yielded greater sensitivity and consistency when compared with swab samples. On 15 August 2020, the US FDA authorized a saliva test developed at Yale University, which gives results in hours. Viral burden measured in upper respiratory specimens declines after symptom onset.

*Isothermal amplification assays*

Isothermal nucleic acid amplification tests also amplify the virus's genome. They are faster than PCR because they don't involve repeated heating and cooling cycles. These tests typically detect DNA using fluorescent tags, which are read out with specialized machines. CRISPR gene editing technology was modified to perform the detection: if the CRISPR enzyme attaches to the sequence, it colors a paper strip. The researchers expect the resulting test to be cheap and easy to use in point-of-care settings. The test amplifies RNA directly, without the RNA-to-DNA conversion step of RT-PCR.

*Antigens*

An antigen is the part of a pathogen that elicits an immune response. Antigen tests look for antigen proteins from the viral surface. In the case of a coronavirus, these are usually proteins from the surface spikes. One of the challenges is to find a target unique to SARS-CoV-2. Isothermal nucleic acid amplification tests can process only one sample at a time per machine. RT-PCR tests are accurate but require too much time, energy, and trained personnel to run the tests. Using these current methods, it is generally believed that there will never be the ability on a PCR test to do 300 million tests a day or to test everybody before they go to work or school.

Samples may be collected via nasopharyngeal swab, a swab of the anterior nares, or from saliva. The sample is then exposed to paper strips containing artificial antibodies designed to bind to coronavirus antigens. Antigens bind to the strips and give a visual readout. The process takes less than 30 minutes, can deliver results at point-of-care, and does not require expensive equipment or extensive training. Swabs of respiratory viruses often lack enough antigen material to be detectable. This is especially true for asymptomatic patients who have little if any nasal discharge. Viral proteins are not amplified in an antigen test. According to the World Health Organization (WHO) the sensitivity of similar antigen tests for respiratory diseases like the flu ranges between 34% and 80%. Based on this information, half or more of COVID-19 infected patients might be missed by such tests, depending on the group of patients tested. While some doubt whether an antigen test can be useful against COVID-19, others have argued that antigen tests are highly sensitive when viral load is high and people are contagious, making them suitable for public health screening. Routine antigen tests can quickly identify when asymptomatic people are contagious, while follow-up PCR can be used if confirmatory diagnosis is needed.

*Imaging*

Typical visible features on chest CT initially include bilateral multilobar ground-glass opacities with a peripheral or posterior distribution. Subpleural dominance, crazy paving, and consolidation may develop as the disease evolves. Chest CT scans and chest x-rays are not recommended for diagnosing COVID-19. Radiologic findings in COVID-19 lack specificity.

*Antibody tests*

The body responds to a viral infection by producing antibodies that help neutralize the virus. Blood tests (serology tests) can detect the presence of such antibodies. Antibody tests can be used to assess what fraction of a population has once been infected, which can then be used to calculate the disease's mortality rate. SARS-CoV-2 antibodies' potency and protective period have not been established. Therefore, a positive antibody test may not imply immunity to a future infection. Further, whether mild or asymptomatic infections produce sufficient antibodies for a test to detect has not been established. Antibodies for some diseases persist in the bloodstream for many years, while others fade away. The most notable antibody classes are IgM and IgG. IgM antibodies are generally detectable several days after initial infection, although levels over the course of infection and beyond are not well characterized. IgG antibodies generally become detectable 10-14 days after infection and normally peak around 28 days after infection. Genetic tests verify infection earlier than antibody tests. Only 30% of those with a positive genetic test produced a positive antibody test on day 7 of their infection.

*Types of Tests*

*Rapid diagnostic test (RDT)*

RDTs typically use a small, portable, positive/negative lateral flow assay that can be executed at point-of-care. RDTs may process blood samples, saliva samples, or nasal swab fluids. RDTs produce colored lines to indicate positive or negative results.

*Enzyme-linked immunosorbent assay (ELISA)*

ELISAs can be qualitative or quantitative and generally require a lab. These tests usually use whole blood, plasma, or serum samples. A plate is coated with a viral protein, such as a SARS-CoV-2 spike protein. Samples are incubated with the protein, allowing any antibodies to bind to it. The antibody- protein complex can then be detected with another wash of antibodies that produce a color/fluorescent readout.

*Neutralization assay*

Neutralization assays assess whether sample antibodies prevent viral infection in test cells. These tests sample blood, plasma, or serum. The test cultures cells that allow viral reproduction (e.g., VeroE6 cells). By varying antibody concentrations, researchers can visualize and quantify how many test antibodies block virus replication.

*Chemiluminescent immunoassay*

Chemiluminescent immunoassays are quantitative lab tests. They sample blood, plasma, or serum. Samples are mixed with a known viral protein, buffer reagents and specific, enzyme-labeled antibodies. The result is luminescent. A chemiluminescent micro-particle immunoassay uses magnetic, protein-coated micro-particles. Antibodies react to the viral protein, forming a complex. Secondary enzyme-labeled antibodies are added and bind to these complexes. The resulting chemical reaction produces light. The radiance is used to calculate the number of antibodies. This test can identify multiple types of antibodies, including IgG, IgM, and IgA.

*Neutralizing vs. binding antibodies*

Most, if not all, large scale COVID-19 antibody testing looks for binding antibodies only and does not measure the more important neutralizing antibodies (NAb). A NAb is an antibody that defends a cell from an infectious particle by neutralizing its biological effects. Neutralization renders the particle no longer infectious or pathogenic. A binding antibody binds to the pathogen but the pathogen remains infective; the purpose can be to flag the pathogen for destruction by the immune system. It may even enhance infectivity by interacting with receptors on macrophages. Since most COVID-19 antibody tests return a positive result if they find only binding antibodies, these tests cannot indicate that the subject has generated protective NAbs that protect against reinfection.

It is expected that binding antibodies imply the presence of NAbs and for many viral diseases total antibody responses correlate somewhat with NAb responses, but this is not established for COVID-19. A study of 175 recovered patients in China who experienced mild symptoms reported that 10 individuals had no detectable NAbs at discharge, or thereafter. How these patients recovered without the help of NAbs and whether they were at risk of re-infection was not addressed. An additional source of uncertainty is that even if NAbs are present, viruses such as HIV can evade NAb responses. Studies have indicated that NAbs to the original SARS virus (the predecessor to the current SARS-CoV-2) can remain active for two years and are gone after six years. Nevertheless, memory cells including Memory B cells and Memory T cells can last much longer and may have the ability to reduce reinfection severity.

*Other tests*

Following recovery, many patients no longer have detectable viral RNA in upper respiratory specimens. Among those who do, RNA concentrations three days following recovery are generally below the range in which replication-competent virus has been reliably isolated. No clear correlation has been described between length of illness and duration of post-recovery shedding of viral RNA in upper respiratory specimens.

*Infectivity*

Infectivity is indicated by the basic reproduction number (R0, pronounced "R naught") of the disease. SARS-CoV-2 is estimated to have an R0 of 2.2 to 2.5. This means that in a population where all individuals are susceptible to infection, each infected person is expected to infect 2.2 to 2.5 others in the absence of interventions. R0 can vary according factors such as geography, population demographics and density. In New York state, R0 was estimated to be 3.4 to 3.8 during its epidemic. On average, an infected person begins showing symptoms five days after infection (the "incubation period") and can infect others beginning two to three days before that. One study reported that 44% of viral transmissions occur within this period. According to CDC, a significant number of infected people who never show symptoms are nevertheless contagious. In vitro studies have not found replication-competent virus after 9 days from infection. The statistically estimated likelihood of recovering replication-competent virus approaches zero by 10 days. Infectious virus has not been cultured from urine or reliably cultured from feces; these potential sources pose minimal if any risk of transmitting infection and any risk can be sufficiently mitigated by good hand hygiene.

Patterns and duration of illness and infectivity have not been fully described. However, available data indicate that SARS-CoV-2 RNA shedding in upper respiratory specimens declines after symptom onset. At 10 days recovery of replication-competent virus in viral culture (as a proxy of the presence of infectious virus) approaches zero. Although patients may produce PCR-positive specimens for up to six weeks, it remains unknown whether these samples hold infectious virus. After clinical recovery, many patients do not continue to shed. Among recovered patients with detectable RNA in upper respiratory specimens, concentrations after three days are generally below levels where virus has been reliably cultured. These data were generated from adults across a variety of age groups and with varying severity of illness. Data from children and infants were not available.

*Nucleic acid tests*

Tests developed in China, France, Germany, Hong Kong, Japan, the United Kingdom, and the US targeted different parts of the viral genome. WHO adopted the German system for manufacturing kits sent to low-income countries without the resources to develop their own tests.

Abbott Laboratories' ID Now nucleic acid test uses isothermal amplification technology. The assay amplifies a unique region of the virus's RdRp gene; the resulting copies are then detected with "fluorescently-labeled molecular beacons". The test kit uses the company's "toaster-size" ID Now device, which is widely deployed in the US. The device can be used in laboratories or in point-of-care settings and provides results in 13 minutes or less.

Primerdesign offers its Genesig Real-Time PCR Coronavirus (COVID-19). Cobas SARS-CoV-2 Qualitative assay runs on the Cobas® 6800/8800 Systems by Roche Molecular Systems. They are offered by the United Nations and other procurement agencies.

*Antigen tests*

Quidel's "Sofia 2 SARS Antigen FIA" is a lateral flow test that uses monoclonal antibodies to detect the virus's nucleocapsid (N) protein. The result is read out by the company's Sofia 2 device using immunofluorescence. The test is simpler and cheaper but less accurate than nucleic acid tests. It can be deployed in laboratories or at point-of-care and gives results in 15 minutes. A false negative result occurs if the sample's antigen level is positive but below the test's detection limit, requiring confirmation with a nucleic acid test.

*Serology (antibody) tests*

Antibodies are usually detectable 14 days after the onset of the infection. Multiple jurisdictions survey their populations using these tests. The test requires a blood draw. Private US labs including Quest Diagnostics and LabCorp offer antibody testing upon request. Antibody tests are available in various European countries. Quotient Limited developed a CE marked COVID-19 antibody test. Roche offers a selective ELISA serology test.

*Sensitivity and specificity*

Sensitivity indicates whether the test accurately identifies whether the virus is present. Each test requires a minimum level of viral load in order to produce a positive result. A 90% sensitive test will correctly identify 90% of infections, missing the other 10% (a false negative). Even relatively high sensitivity rates can produce high rates of false negatives in populations with low incidence rates.

Specificity indicates how well-targeted the test is to the virus in question. Highly specific tests pick up only the virus in question. Non-selective tests pick up other viruses as well. A 90% specific test will correctly identify 90% of those who are uninfected, leaving 10% with a false positive result. Low- specificity tests have a low positive predictive value (PPV) when prevalence is low. For example, suppose incidence is 5%. 100 people selected at random would contain 95 people who are negative and 5 people who are positive. Using a test that has a specificity of 95% would yield on average 4.75 people who are actually negative who would incorrectly test positive. If the test has a sensitivity of 100%, all five positive people would also test positive, totaling 9.75 positive results. Thus, the PPV is 51.3%, an outcome comparable to a coin toss. In this situation retesting those with a positive result increases the PPV to 95.5%, meaning that only 4.5% of the second tests would return the incorrect result, on average less than 1 incorrect result.

*Causes of test error*

Improper sample collection is exemplified by failure to acquire enough sample and failure to insert a swab deep into the nose. This results in insufficient viral load, one cause of low clinical sensitivity. The time course of infection also affects accuracy. Samples may be collected before the virus has had a chance to establish itself or after the body has stopped its progress and begun to eliminate it. Improper storage for too long a time can cause RNA breakdown and lead to wrong results as viral particles disintegrate. Improper design and manufacturing can yield inaccurate results. Millions of tests made in China were rejected by various countries throughout the period of March 2020 through May 2020. Test makers typically report the accuracy levels of their tests when seeking approval from authorities. In some jurisdictions, these results are cross-validated by additional assessments. Reported results may not be achieved in clinical settings due to such operational inconsistencies.

*PCR-based test*

RT-PCR is the most accurate diagnostic test. It typically has high sensitivity and specificity in a laboratory setting: however, in one study sensitivity dropped to 66-88% clinically. In one study sensitivity was highest at week one (100%), followed by 89.3%, 66.1%, 32.1%, 5.4% and zero by week six. A Dutch CDC-led laboratory investigation compared 7 PCR kits. Test kits made by BGI, R-Biopharm AG, BGI, KH Medical and Seegene showed high sensitivity. High sensitivity kits are recommended to assess people without symptoms, while lower sensitivity tests are adequate when diagnosing symptomatic patients.

*Isothermal nucleic amplification test*

One study reported that the ID Now COVID-19 test showed sensitivity of 85.2%. Abbott responded that the issue could have been caused by analysis delays. Another study rejected the test in their clinical setting because of this low sensitivity.

What is needed is an apparatus and method for point-of-care, rapid, field-deployable diagnostic testing of Covid-19, viruses, antibodies and markers, which can be used by unskilled health workers, which is sensitive and specific, and which gives diagnostic results in 30 minutes or less with highly developed diagnostic data processing in the Cloud.

According to an aspect, the present invention provides an automated system communicating with a remote server for diagnostically field testing a sample taken from a subject using an automated portable handheld instrument to determine the presence of viral antigens and/or antibodies thereto comprising: one or more types of microfluidic circuits defined in a rotatable disk, each type of microfluidic disk for performing a bioassay using a predetermined type of bio-detector to generate an electrical signal indicative of a bioassay measurement; a bio-detector operationally positioned in the microfluidic circuit; one or more lasers; one or more positionable valves in the microfluidic circuit; and a backbone unit for rotating the disk according to a predetermined protocol to perform the bioassay, for controlling and powering the one or more lasers to selectively open one or more positionable valves in the microfluidic disk, for operating the bio-detector to generate an electrical signal indicative of a bioassay measurement; for communicating the bioassay measurement to the remote server, and for associating the performed bioassay and its corresponding bioassay measurement to the subject.

The bio-detector may comprise a microarray and the bioassay may be a serology test that may include testing for IgG and/or IgM. The bio-detector may comprise a microarray and the serology test provided by the microarray may be a respiratory antibody and/or antigen test. The serology test may test for Covid-19.

The bio-detector may comprise a microarray and the microfluidic disk may have a center and comprise: a sample inlet; a blood-plasma separation chamber communicated with the sample inlet and preferably positioned on the disk radially farther from the center of the disk than the sample inlet; a mixing chamber communicated to the blood-plasma separation chamber through a corresponding selectively openable valve and preferably positioned on the disk radially farther from the center of the disk than the blood-plasma separation chamber; a first wash chamber communicated to the mixing chamber through a corresponding selectively openable valve and preferably positioned on the disk radially closer to the center of the disk than the mixing chamber; a secondary antibody chamber communicated to the mixing chamber through a corresponding selectively openable valve and preferably positioned on the disk radially closer to the center of the disk than the mixing chamber; a second wash chamber communicated to the mixing chamber through a corresponding selectively openable valve and preferably positioned on the disk radially closer to the center of the disk than the mixing chamber; a microarray chamber communicated to the mixing chamber, the microarray being disposed in the microarray chamber; and the microarray chamber preferably positioned on the disk radially farther from the center of the disk than the mixing chamber; and a waste chamber communicated to the microarray chamber by a siphon and by a corresponding selectively openable spin-dry valve and preferably positioned on the disk radially farther from the center of the disk than the microarray chamber.

The signal indicative of a bioassay measurement may be a digital image of microarray spots that have been fluoroscopically activated by the sample in the performance of the bioassay. The remote server can be a Cloud server. The backbone unit may include network circuitry which communicates the digital image to the Cloud server and a corresponding schema file associating the subject to the performed bioassay and its corresponding bioassay measurement. The Cloud server, operating in an automated and modular protocol, may align the microarray spots of the digital image, detect each of the aligned spots of the microarray and analyze each of the spots of the digital image to assign a scalar value to each microarray spot to produce a processed microarray measurement set of data. The Cloud server, operating in an automated protocol, may analyze the processed microarray measurement set of data to produce a diagnosis of the bio-measurement. The Cloud server, operating in an automated protocol, may report the results to the subject as determined by the schema file.

The Cloud server may comprise a cloud-based module for automatically determining under automated control whether the corresponding Z- scores of the communicated data output of positive and/or negative indications are indicative of Covid-19 rather than the Z-scores of the plurality of viral infections sharing at least some of the Covid-19 antigens and/or antibodies.

The Cloud server may comprise means for identifying positive and/or negative indications of the digital image of microarray spots for a plurality of acute respiratory infections selected from the group including SARS-CoV-2, SARS-CoV, MERS-CoV, common cold coronaviruses (HKU1 , OC43, NL63, 229E), and multiple subtypes of influenza, adenovirus, metapneumovirus, parainfluenza, and/or respiratory syncytial virus.

The Cloud server may comprise a cloud-based module for automatically evaluating antigens to discriminate output data of a positive group of antigens from a negative group of antigens across a range of assay cutoff values using receiver-operating-characteristic (ROC) curves for which an area- under curve (AUC) is measured to determine high performing antigens to diagnose Covid-19.

The Cloud server may comprise a cloud based module for automatically determining under automated control an optimal sensitivity and specificity for Covid-19 from a combination of a plurality of high performing antigens based on a corresponding Youden Index calculated for the combination of plurality of high-performing antigens.

According to an aspect, the invention provides an automated system communicated to a cloud-based server for diagnostically field testing a sample taken from a subject using an automated portable handheld instrument to determine the presence of viral antigens and/or antibodies thereto in a serology test to detect Covid-19. The system includes: a microfluidic circuit defined in a rotatable disk for performing a bioassay using a microarray to generate an digital image indicative of a bioassay measurement; a microarray operationally positioned in the microfluidic circuit; one or more positionable valves in the microfluidic circuit; one or more lasers; a fluoroscopic microarray reader; and a backbone unit for rotating the disk according to a predetermined protocol to perform the bioassay, for controlling and powering the one or more lasers to selectively open one or more positionable valves in the microfluidic disk, for operating the fluoroscopic microarray reader to generate the digital image indicative of a bioassay measurement; for communicating the digital image to the cloud-based server, and for associating the performed bioassay and its corresponding bioassay measurement to the subject. The microfluidic disk has a center and comprises: a sample inlet; a blood-plasma separation chamber communicated with the sample inlet and preferably positioned on the disk radially farther from the center of the disk than the sample inlet; a mixing chamber communicated to the blood-plasma separation chamber through a corresponding selectively openable valve and preferably positioned on the disk radially farther from the center of the disk than the blood-plasma separation chamber; a first wash chamber communicated to the mixing chamber through a corresponding selectively openable valve and preferably positioned on the disk radially closer to the center of the disk than the mixing chamber; a secondary antibody chamber communicated to the mixing chamber through a corresponding selectively openable valve and preferably positioned on the disk radially closer to the center of the disk than the mixing chamber; a second wash chamber communicated to the mixing chamber through a corresponding selectively openable valve and preferably positioned on the disk radially closer to the center of the disk than the mixing chamber; a microarray chamber communicated to the mixing chamber, the microarray being disposed in the microarray chamber; and the microarray chamber preferably positioned on the disk radially farther from the center of the disk than the mixing chamber; and a waste chamber communicated to the microarray chamber by a siphon and by a corresponding selectively openable spin-dry valve and preferably positioned on the disk radially farther from the center of the disk than the microarray chamber. The backbone unit includes network circuitry which communicates the digital image to the Cloud server and a corresponding schema file associating the subject to the performed bioassay and its corresponding bioassay measurement. The Cloud server, operating in an automated and modular protocol, aligns the microarray spots of the digital image, detects each of the aligned spots of the microarray and analyzes each of the spots of the digital image to assign a scalar value to each microarray spot to produce a processed microarray measurement set of data. The Cloud server, operating in an automated protocol, analyzes the processed microarray measurement set of data to produce a diagnosis of the bio-measurement. The Cloud server, operating in an automated protocol, reports the results to the subject as determined by the schema file.

The Cloud server may comprise a cloud-based module for automatically determining under automated control whether the corresponding Z- scores of the communicated data output of positive and/or negative indications are indicative of Covid-19 rather than the Z-scores of the plurality of viral infections sharing at least some of the Covid-19 antigens and/or antibodies.

The Cloud server may comprise means for identifying positive and/or negative indications of the digital image of microarray spots for a plurality of acute respiratory infections selected from the group including SARS-CoV-2, SARS-CoV, MERS-CoV, common cold coronaviruses (HKU1 , OC43, NL63, 229E), and multiple subtypes of influenza, adenovirus, metapneumovirus, parainfluenza, and/or respiratory syncytial virus.

The Cloud server may comprise a cloud-based module for automatically evaluating antigens to discriminate output data of a positive group of antigens from a negative group of antigens across a range of assay cutoff values using receiver-operating-characteristic (ROC) curves for which an area-under curve (AUC) is measured to determine high performing antigens to diagnose Covid-19.

The Cloud server may comprise a cloud based module for automatically determining under automated control an optimal sensitivity and specificity for Covid-19 from a combination of a plurality of high performing antigens based on a corresponding Youden Index calculated for the combination of plurality of high-performing antigens.

According to a further aspect, the present invention provides a method for operating an automated system communicated to a remote server for diagnostically field testing a sample taken from a subject using an automated portable handheld instrument to determine the presence of viral antigens and/or antibodies thereto comprising the steps of: introducing the sample into a sample inlet; transferring the sample to a blood-plasma separation chamber communicated with the sample inlet and preferably positioned on the disk radially farther from the center of the disk than the sample inlet; separating the blood from the plasma by spinning the disk, preferably at 5500 rpm for 5 minutes; opening a first valve using a laser-meltable plug, the first valve being disposed in a conduit in the disk between the blood-plasma chamber and a mixing chamber communicated to the blood-plasma separation chamber through the selectively openable first valve and preferably positioned on the disk radially farther from the center of the disk than the blood-plasma separation chamber; transferring the serum to the mixing chamber and to a microarray chamber communicated to the mixing chamber, the microarray being disposed in the microarray chamber; and the microarray chamber preferably positioned on the disk radially farther from the center of the disk than the mixing chamber; reciprocating the sample in the microarray chamber, preferably for 40 cycles at 2700-5428 rpm, followed by priming, preferably at 170 rpm and evacuation at 1000 rpm for 5 minutes, to a waste chamber communicated to the microarray chamber by a siphon and by a corresponding selectively openable spin-dry valve and preferably positioned on the disk radially farther from the center of the disk than the microarray chamber; opening a second valve using a laser-meltable plug, the second valve being disposed in a conduit in the disk between the mixing chamber and a first wash chamber communicated to the mixing chamber through a corresponding selectively openable valve and preferably positioned on the disk radially closer to the center of the disk than the mixing chamber; transferring a first wash from the first wash chamber through the mixing chamber to the microarray chamber; reciprocating the first wash in the microarray chamber, preferably for 20 cycles at 2700-5428 rpm, followed by priming, preferably at 170 rpm, and evacuation, preferably at 1000 rpm for 2 minutes, to the waste chamber; opening a third valve using a laser-meltable plug, the third valve being disposed in a conduit in the disk between the mixing chamber and a secondary antibody chamber communicated to the mixing chamber through a corresponding selectively openable valve and preferably positioned on the disk radially closer to the center of the disk than the mixing chamber; transferring the secondary antibody from the secondary antibody chamber through the mixing chamber to the microarray chamber; reciprocating the secondary antibody in the microarray chamber, preferably for 20 cycles at 2700-5428 rpm, followed by priming, preferably at 170 rpm, and evacuation, preferably at 1000 rpm for 2 minutes, to the waste chamber; opening a fourth valve using a laser-meltable plug, the fourth valve being disposed in a conduit in the disk between the mixing chamber and a second wash chamber communicated to the mixing chamber through a corresponding selectively openable valve and preferably positioned on the disk radially closer to the center of the disk than the mixing chamber; transferring a second wash from the second wash chamber through the mixing chamber to the microarray chamber; reciprocating the second wash in the microarray chamber, preferably for 20 cycles at 2700-5428 rpm, followed by priming, preferably at 170 rpm, and evacuation, preferably at 1000 rpm for 2 minutes, to the waste chamber; opening a fifth valve using a laser-meltable plug, the fifth valve being disposed in a conduit in the disk between the microarray chamber and the waste chamber; spin drying the microarray chamber by spinning the disk, preferably at 5500 rpm for one minute; moving the microarray chamber to a position wherein a fluoroscopically induced digital image can be taken of the microarray; and generating the fluoroscopically induced digital image of the microarray.

The method may further comprise the steps of: communicating the digital image using a backbone unit including network circuitry which communicates the digital image to a Cloud server and communicates a corresponding schema file associating the subject to the performed bioassay and its corresponding bioassay measurement; aligning the microarray spots of the digital image in the Cloud server, operating in an automated and modular protocol; detecting each of the aligned spots of the microarray in the Cloud server, operating in an automated and modular protocol; analyzing each of the spots of the digital image in the Cloud server, operating in an automated and modular protocol to assign a scalar value to each microarray spot to produce a processed microarray measurement set of data; analyzing the processed microarray measurement set of data to produce a diagnosis of the bio-measurement in the Cloud server, operating in an automated protocol; and reporting the results to the subject as determined by the schema file using the Cloud server, operating in an automated protocol.

The step of analyzing the processed microarray measurement set of data may comprise the step of identifying positive and/or negative indications of the digital image of microarray spots for a plurality of acute respiratory infections selected from the group including SARS-CoV-2, SARS-CoV, MERS-CoV, common cold coronaviruses (HKU1 , OC43, NL63, 229E), and multiple subtypes of influenza, adenovirus, metapneumovirus, parainfluenza, and/or respiratory syncytial virus.

According to a further aspect, the present invention provides an automated system communicated to a remote server for diagnostically field testing a sample taken from a subject using an automated portable handheld instrument to determine the presence of viral antigens and/or antibodies thereto, the system having a microfluidic disk.

The microfluidic disk comprises a sample inlet, a blood-plasma separation chamber communicated with the sample inlet and preferably positioned on the disk radially farther from the center of the disk than the sample inlet, the blood-plasma separation chamber being configured for separating the blood from the plasma by spinning the disk.

The disk further comprises a mixing chamber communicated to the blood-plasma separation chamber, and preferably positioned on the disk radially farther from the center of the disk than the blood-plasma separation chamber, and a first conduit in the disk between the blood-plasma chamber and the mixing chamber.

The disk also comprises a selectively operable first valve disposed in the first conduit, a microarray chamber communicated to the mixing chamber and preferably positioned on the disk radially farther from the center of the disk than the mixing chamber, wherein the automated system is configured to selectively transfer separated serum from the mixing chamber into the microarray chamber.

Additionally, the disk comprises a microarray having a plurality of microarray spots, the microarray disposed in the microarray chamber, and a waste chamber preferably positioned on the disk radially farther from the center of the disk than the microarray chamber. The disk further comprises a second conduit selectively communicating the microarray chamber with the waste chamber, a selectively operable second valve disposed in the second conduit, a siphon communicating the microarray chamber to the waste chamber, a third conduit communicated with the mixing chamber, a first wash chamber, a selectively operable third valve communicated between the first wash chamber and the third conduit, a secondary antibody chamber, a selectively operable fourth valve communicated between the antibody chamber and the third conduit, a second wash chamber, and a selectively operable fourth valve communicated between the second wash chamber and the third conduit.

The blood-plasma separation chamber may be configured to separate the blood from the plasma by spinning the disk, preferably at 5500 rpm for 5 minutes. Additionally or alternatively, the automated system may be configured to reciprocate the sample in the microarray chamber, preferably for 40 cycles at 2700-5428 rpm, followed by priming, preferably at 170 rpm, and evacuation, preferably at 1000 rpm for 5 minutes, to the waste chamber communicated to the microarray chamber by the siphon and by the selectively openable second valve.

Further to the above, the automated system may be configured to selectively transfer a first wash from the first wash chamber through the third valve through the mixing chamber to the microarray chamber. In addition, the automated system can be configured to reciprocate the first wash in the microarray chamber, preferably for 20 cycles at 2700-5428 rpm, followed by priming, preferably at 170 rpm, and evacuation, preferably at 1000 rpm for 2 minutes, to the waste chamber through the second valve.

The automated system can be configured to selectively operate the fourth valve to selectively transfer a secondary antibody from the secondary antibody chamber through the third conduit to the mixing chamber. Furthermore, the automated system can be configured to, upon selectively transferring the secondary antibody from the secondary antibody chamber through the third conduit to the mixing chamber, transfer the secondary antibody through the mixing chamber to the microarray chamber. In this case, the automated system can be configured to reciprocate the secondary antibody in the microarray chamber, preferably for 20 cycles at 2700-5428 rpm, followed by priming, preferably at 170 rpm, and evacuation, preferably at 1000 rpm for 2 minutes, to the waste chamber through the second valve.

The automated system can be configured to selectively transfer a second wash through the fourth valve through the third conduit to the mixing chamber to the microarray chamber. In this case, the automated system can be configured to reciprocate the second wash in the microarray chamber, preferably for 20 cycles at 2700-5428 rpm, followed by priming, preferably at 170 rpm, and evacuation, preferably at 1000 rpm for 2 minutes, to the waste chamber through the second valve. In this case, the automated system can be configured to spin dry the microarray chamber by spinning the disk, preferably at 5500 rpm for one minute, wherein the automated system can be configured to move the microarray chamber to a position wherein a fluoroscopically induced digital image can be taken of the microarray.

The automated system may further comprise a Cloud server, and a backbone unit including network circuitry configured for communicating the digital image to the Cloud server and for communicating a corresponding schema file to thereby associate the subject to the performed bioassay and its corresponding bioassay measurement. In this case, the Cloud server can be configured for aligning the microarray spots of the digital image, for detecting each of the aligned spots of the microarray in the Cloud server, and for analyzing each of the spots of the digital image. Furthermore, the Cloud server can be configured for assigning a scalar value to each microarray spot to produce a processed microarray measurement set of data, for analyzing the processed microarray measurement set of data to produce a diagnosis of the bio-measurement in the Cloud server, and for reporting the results to the subject as determined by the schema file using the Cloud server, all the while operating in an automated and modular protocol.

According to a further aspect, the present invention provides a method of coordinating user flow of an automated system communicated to a remote server for diagnostically field testing a sample taken from a patient using an automated portable handheld instrument to determine the presence of viral antigens and/or antibodies using a rotatable disk of a given type among a plurality of rotatable disks of one or more types, wherein a microfluidic circuit is defined in each rotatable disk, each rotatable disk of a given type being configured for performing a bioassay using a predetermined type of bio-detector disposed in the microfluidic disk, and in which a backbone unit is used for rotating a rotatable disk among the plurality of rotatable disks according to a predetermined protocol to perform the corresponding bioassay, for operating the corresponding bio-detector to generate an electrical signal indicative of a bioassay measurement, for communicating the bioassay measurement to the remote server, and for associating the performed bioassay and its corresponding bioassay measurement to the patient, the method coordinating tasks between the patient, the portable handheld instrument, the remote server, and a test operator of the portable handheld instrument.

The method comprises logging into a portal to schedule an automated diagnostic test at a location by the patient, automatically scheduling the test and generating a unique QR privacy and control code whereby the patient controls communication of all test results, the unique QR privacy and control code identifying the patient, the test time and location, and the type of rotatable disk to be used in the bioassay.

The method further comprises automatically communicating the unique QR privacy and control code to the patient, automatically communicating appointment information for the patient to the test operator, presenting the unique QR privacy and control code by the patient to the test operator at the test location and sending the unique QR privacy and control code to the remote server, automatically determining if the unique QR privacy and control code is valid in remote server, and automatically contingently authorizing the test in a designated type of rotatable disk for a corresponding bioassay.

The method also includes loading a rotatable disk of the designated type among the plurality of rotatable disks into the portable handheld instrument by the test operator with a verified scanning of a code on the disk to confirm the designated type of disk, communicating the scanned code to the remote server, automatically checking the scanned code of the disk loaded into the portable handheld instrument in the remote server, and if correct, downloading metadata of the disk from the remote server to the portable handheld instrument, and taking a specimen from the patient and loading the specimen into the disk by the test operator.

The method further comprises initiating the automated test by the test operator in the portable handheld instrument, automatically performing the bioassay using the disk in the portable handheld instrument to generate a digital data result of the bioassay, automatically communicating the digital data result of the bioassay to the remote server, automatically data processing the digital data result of the bioassay in the remote server to generate a predictive diagnostic analysis, and automatically communicating the predictive diagnostic analysis from the remote server to a patient-controlled device.

The method may further comprise communicating the predictive diagnostic analysis from the patient-controlled device to others only with presentation of the unique QR privacy and control code.

The bioassay may be performed using a microarray as the bio-detector in the rotatable disk. Automatically performing the bioassay using the disk in the portable handheld instrument to generate a digital data result of the bioassay may comprise performing a pre-test diagnostic of the microarray to determine that at least three fiducials are visible, that fiducial intensity is within 20% of original images, and that fiducials are in focus by a data camera in the portable handheld instrument.

Automatically communicating the predictive diagnostic analysis from the remote server to a patient-controlled device may comprise automatically generating a prediction and a corresponding confidence interval.

The remote server may be a Cloud-based server.

According to a further aspect, the present invention provides an automated system for coordinating medical field testing and data acquisition that comprises a plurality of rotatable disks of one or more types, wherein a microfluidic circuit is defined in each rotatable disk, each rotatable disk of a given type being configured for performing a bioassay using a predetermined type of bio-detector disposed in the microfluidic disk. The system additionally comprises an automated portable handheld instrument to determine the presence of viral antigens and/or antibodies using the microfluidic circuit of a rotatable disk of a given type among the plurality of rotatable disks that is loaded in the automated portable handheld instrument, wherein the automated portable handheld instrument is configured for operating the disk according to a predetermined protocol to generate an electrical signal indicative of a bioassay measurement, and for associating the performed bioassay and its corresponding bioassay measurement to the patient. The system further comprises a remote server for diagnostically analyzing results of field testing a sample taken from a patient communicated with the automated portable handheld instrument to diagnostically analyze a sample taken from a patient, wherein the remote server has a portal to schedule an automated diagnostic test at a location by the patient.

The remote server is configured to automatically schedule the test and to generate a unique QR privacy and control code whereby the patient controls communication of all test results, the unique QR privacy and control code identifying the patient, the test time and location, and the type of rotatable disk to be used in the bioassay, wherein the remote server is configured to automatically communicate the unique QR privacy and control code to the patient, and wherein the remote server is configured to automatically communicate appointment information for the patient to the test operator.

The portable handheld instrument is configured to scan the unique QR privacy and control code presented by the patient to the test operator at the test location and to send the unique QR privacy and control code to the remote server, wherein the remote server is configured to automatically determine if the unique QR privacy and control code is valid, and to automatically contingently authorize the test in a designated type of disk for a corresponding bioassay.

The portable handheld instrument is configured to, after the test operator has performed a verified scanning of a code on the disk to confirm the designated type of disk, communicate the scanned code to the remote server, wherein the remote server is configured to automatically check the scanned code of the disk loaded into the portable handheld instrument, and if correct, to download metadata from the remote server to the portable handheld instrument.

The portable handheld instrument is configured to automatically perform the bioassay using the disk to generate a digital data result of the bioassay when a specimen taken from the patient is loaded into the disk by the test operator and the automated test is initiated by the test operator, wherein the portable handheld instrument is configured to automatically communicate the digital data result of the bioassay to the remote server, wherein the remote server is configured to automatically data process the digital data result of the bioassay to generate a predictive diagnostic analysis, and wherein the remote server is configured to automatically communicate the predictive diagnostic analysis to a patient-controlled device.

The remote server can be configured to communicate the predictive diagnostic analysis from the patient-controlled device to others only with presentation of the unique QR privacy and control code. Additionally or alternatively, the microfluidic circuits of the rotatable disks may each comprise a microarray, wherein the portable handheld instrument may include a data camera, and wherein the portable handheld instrument may generate a digital data result of the bioassay by performing a pre-test diagnostic of the microarray to determine that at least three fiducials are visible, that fiducial intensity is within 20% of original images, and that fiducials are in focus by the data camera.

The remote server can be configured to automatically communicate the predictive diagnostic analysis to a patient-controlled device by automatically generating a prediction and a corresponding confidence interval.

According to a further aspect, the present invention provides a system for an automated diagnostic procedure in combination with a patient-controlled device comprising a unique privacy code, capable of storage in a tangible medium, identifying a patient and a field portable medical assay performed on the patient, the use of which code controls access to any communication relating to the patient and a bioassay, and to the use and privacy of medical data relating to the patient and bioassay and to a related diagnosis. The system further comprises a mobile field device for performing a laboratory quality assay in a microfluidic disk of a specimen from the patient in which disk a surface acoustic wave (SAW) detector for direct measure of a virus, bacterium, fungus or biomarker, an antibody microarray for measure of human antibody immunological response, and/or a reverse transcription - polyclonal repetition (RT-PCR) photometric detector for direct RNA detection of a virus is employed, where the mobile field device is capable of use by an operator without necessary specialized medical training to perform the field portable bioassay, and where the mobile field device generates the medical data without diagnostic processing the medical data in the mobile field device. The system further comprises a Cloud-based remote server to receive communications from the mobile field device to automatically store and automatically process and analyze the medical data from the mobile field device in association with the unique code identifying the patient to generate a predictive diagnosis without human intervention, the Cloud-based remote server automatically communicating to a patient-controlled device the predicative diagnosis and any related medical analysis information for further recommunication to patient-selected physicians, healthcare provides, governmental units and/or others selected by the patient.

According to a further aspect, the present invention provides a method for an automated diagnostic procedure related to a bioassay in combination with a patient-controlled device comprising generating a unique privacy code, capable of storage on or in a tangible medium, identifying a patient and a field portable medical assay performed on the patient, in association with the unique privacy code, the use of which controls access to any communication relating to the patient and the bioassay, and relating to the use and privacy of medical data of the patient, the bioassay and any related diagnosis.

The method further comprises using a mobile field device for performing a laboratory quality assay of a specimen from the patient in a microfluidic disk in which disk a surface acoustic wave (SAW) detector for direct measure of a virus, bacterium, fungus or biomarker, an antibody microarray for measure of human antibody immunological response, and/or a reverse transcription - polyclonal repetition (RT-PCR) photometric detector for direct RNA detection of a virus is employed, wherein the mobile field device is capable of use by an operator without necessary specialized medical training to perform the field portable bioassay, and wherein the mobile field device generates the medical data without diagnostic processing the medical data in the mobile field device.

The method additionally comprises receiving communications from the mobile field device in a remote server to automatically store and automatically process and analyze the medical data from the mobile field device in association with the unique code identifying the patient to generate a predictive diagnosis without human intervention, and automatically communicating from the remote server to a patient-controlled device the predicative diagnosis and any related medical analysis information for further recommunication to patient-selected physicians, healthcare provides, governmental units and/or others as selected by the patient.

According to a further aspect, the present invention provides a method of data chain identification communicated to a remote Cloud-based server for diagnostically field testing a sample taken from a subject using an automated portable handheld instrument to determine the presence of viral antigens and/or antibodies thereto, the data chain identification included in an image file of an assay of the viral antigens and/or antibodies performed in a microfluidic disk that is loaded in the automatic portable handheld instrument and that includes a microarray.

The method comprises providing the data chain identification structured as a tree graph including recursively accessible nodes to a unique patient/test code, a unique machine ID, a unique cartridge code, a UTC timestamp of the assay, and a unique cartridge code, wherein the machine ID is uniquely defined by a camera serial number and on-board computer (pi raspberry) serial number, wherein the cartridge code is defined by a cartridge assembly batch, which details a date of assembly, microarray information, disc information, and reagent catalog and lot number, wherein the disc information defined by a disc design and disc injection batch, wherein the microarray information is defined by a printing date, a microarray layout, a glass slide etching batch, a printing protein catalog and lot number, and a nitrocellulose lot used in the microarray, and wherein the glass slide etching batch is defined by a glass slide lot.

According to a further aspect, the present invention provides a remote Cloud-based system for diagnostically field testing a sample taken from a subject comprising a Cloud-based server, and an automated portable handheld instrument to determine the presence of viral antigens and/or antibodies thereto using a microfluidic disk that includes a microarray and that is loaded into the automated portable handheld instrument. The automated portable handheld instrument is configured to generate an image file of an assay of the viral antigens and/or antibodies performed in the portable handheld instrument using the microfluidic disk, and to send the image file to the Cloud-based server, wherein the image file includes a data chain identification allowing control of data sent to the Cloud-based server. The data chain identification is structured as a tree graph including recursively accessible nodes to a unique patient/test code, a unique machine ID, a unique cartridge code, a UTC timestamp of the assay, and a unique cartridge code, wherein the machine ID is uniquely defined by a camera serial number and on-board computer serial number, the camera and on-board computer being included in the automated portable handheld instrument.

The cartridge code is defined by a cartridge assembly batch, which details a date of assembly, microarray information, disk information, and reagent catalog and lot number, wherein the disk information defined by a disk design and disk injection batch, wherein the microarray information is defined by a printing date, a microarray layout, a glass slide etching batch, a printing protein catalog and lot number, and a nitrocellulose lot used in the microarray, and wherein the glass slide etching batch is defined by a glass slide lot.

According to a further aspect, the present invention provides a Cloud-based server that is suitable for a remote Cloud-based system as defined above, wherein the Cloud-based server is configured for controlling data sent to the Cloud-based server from an automated portable handheld instrument of the remote Cloud-based system using the data chain identification included in the image file generated by the automated portable handheld instrument.

The disclosure can be better visualized by turning now to the following drawings wherein like elements are referenced by like numerals.

Fig. 1 is a front three-quarter perspective of the backbone unit.

Fig. 2 is the view of Fig. 1 with the disk lid opened.

Fig. 3 is an end plan view of the backbone unit showing the external connections.

Fig. 4 is a front three-quarter perspective of the backbone unit with the cover removed showing the major components included in the backbone unit.

Fig. 5 is a block diagram of the circuitry and elements in the backbone unit.

Fig. 6 is a diagram of an LED ring board to provide even IR activation illumination to the sample in the microarray chamber.

Fig. 7 is a top plan view of the microfluidic disk carrying a microarray.

Fig. 8 is a flow diagram of the operation of the disk of Fig. 7.

Fig. 9 is flow diagram of the workflow implemented by the backbone unit.

Fig. 10 is a block diagram of the software architecture used to implement the workflow of Fig. 9.

Fig. 11 is a screenshot of the operator interface.

Fig. 12 is a screenshot of the operator interface when Scan QR Code is chosen in Fig. 11.

Fig. 13 is a block diagram of the backend operation architecture of the software operating in the backbone unit.

Fig. 14 is a flow diagram of the digital image analysis performed by the Cloud server.

Fig. 15 is a block diagram of the software organization used to implement the flow diagram of Fig. 14.

Figs. 16a and 16b are graphs showing the IgG seroreactivity as measured by means of the fluorescence intensity of serum specimens on the coronavirus antigen microarray. Fig. 16a is a graph of fluorescence values from antigen spots specific to a plurality of viruses, shown side by side. Fig. 16b is an enlargement of SARS-CoV-2, SARS-CoV and MERS-CoV antigen spots.

Fig. 17 is a graph of normalized IgG reactivity of positive and negative sera on coronavirus antigen microarray. The plot shows IgG reactivity against each antigen measured as mean fluorescence intensity (MFI) with full range (bars) and interquartile range (boxes) for convalescent sera from PCR- positive individuals (positive, red) and sera from nine individuals prior to pandemic (negative, blue). Below the plot, the heatmap shows average reactivity for each group (white::: low, black::: mid, red::: high). The antigen labels are color coded for respiratory virus group.

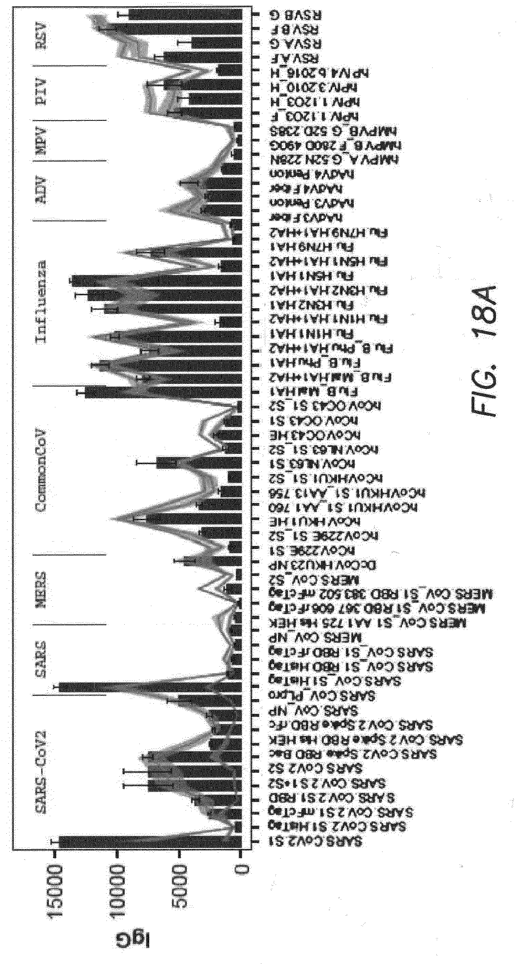

Figs. 18a -18d are graphs of an individual patient's fluorescence values for IgG and IgM detection in a sample using a microarray, and the corresponding Z-score statistics. The red line is an average positive result used to assess whether a measure is positive. The blue line is an average of negative results. The red corresponds to an average seropositive result which is additionally confirmed via PCR. The blue line corresponds to an average seronegative result which is confirmed via PCR. If a patient's IgG bar graph looks like the red line, they test positive, if it looks like the blue line, they test negative.

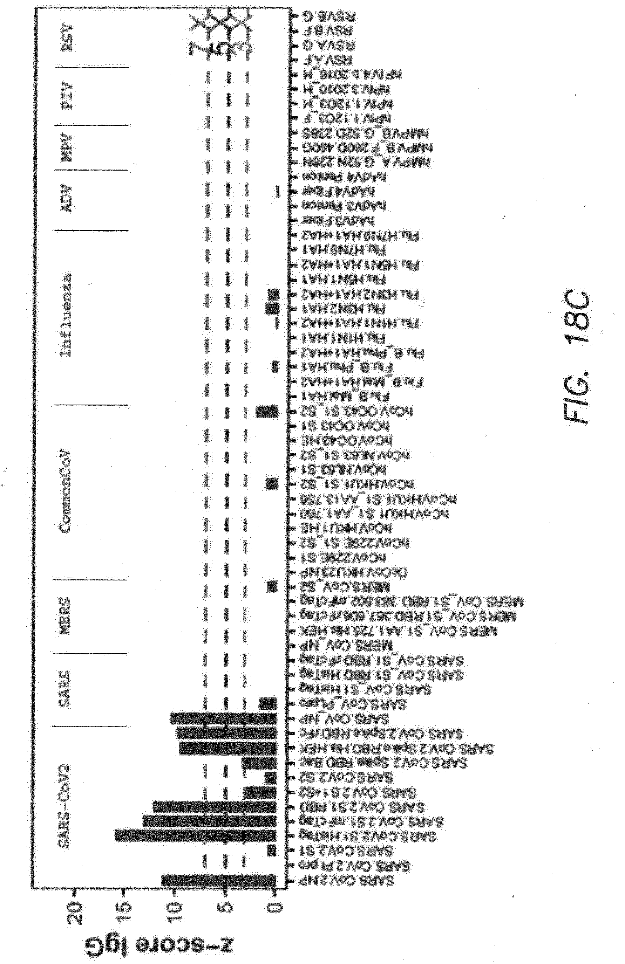

Fig. 18a is a graph of the fluorescence values for IgG for several viruses, namely SARS-CoV2, SARS, MERS, CommonCoV, Influenza, ADV, MPV, PIV and RSV as a function of the antigen spots on the microarray as seen as listed on the x-axis in Fig. 18c.

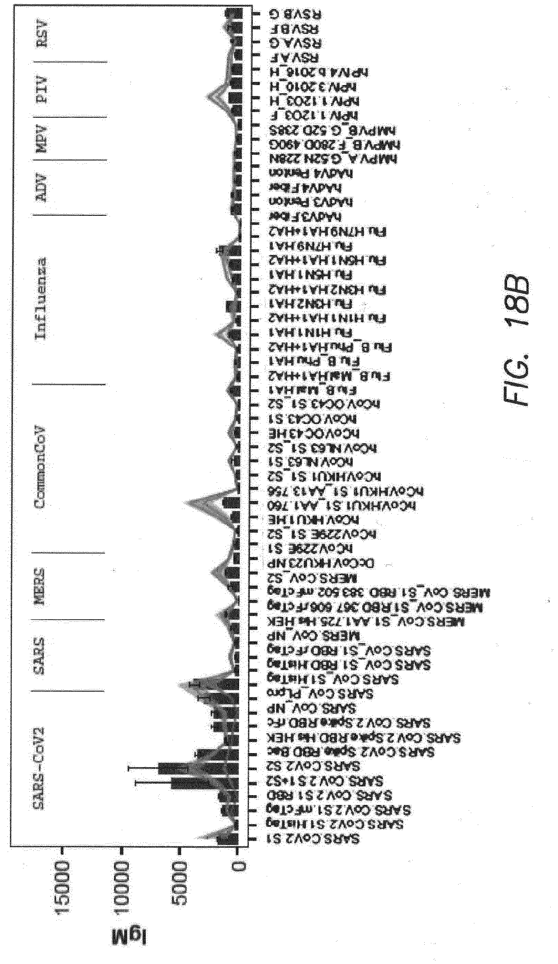

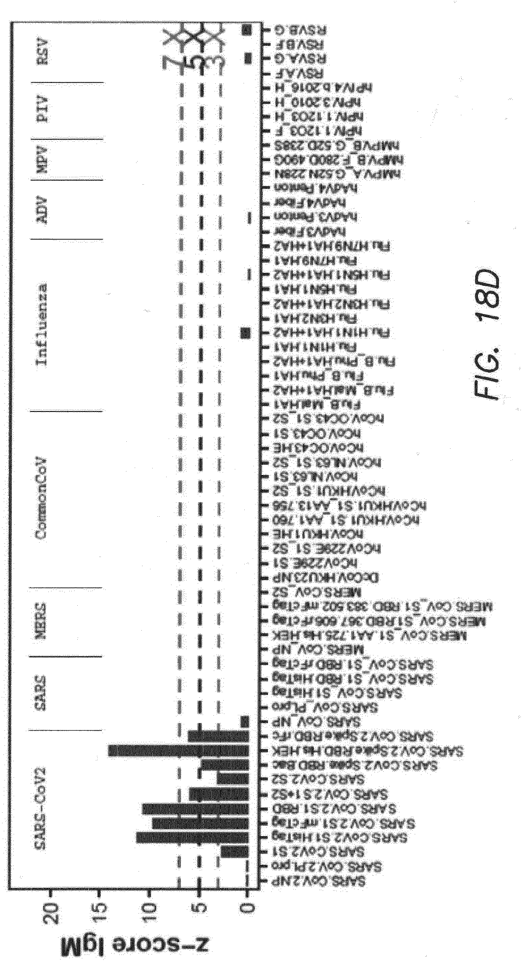

Fig. 18b is a graph of the fluorescence values for IgM for several viruses, namely SARS-CoV2, SARS, MERS, CommonCoV, Influenza, ADV, MPV, PIV and RSV as a function of the antigen spots on the microarray as seen as listed on the x-axis in Fig. 18d.

Fig. 18c is a bar graph of the Z-score statistics of the IgG readings for several viruses, namely SARS-CoV2, SARS, MERS, CommonCoV, Influenza, ADV, MPV, PIV and RSV as a function of the antigen spots on the microarray as listed on the x-axis.

Fig. 18d is a bar graph of the Z -score statistics of the IgM readings for several viruses, namely SARS-CoV2, SARS, MERS, CommonCoV, Influenza, ADV, MPV, PIV and RSV as a function of the antigen spots on the microarray as listed on the x-axis.

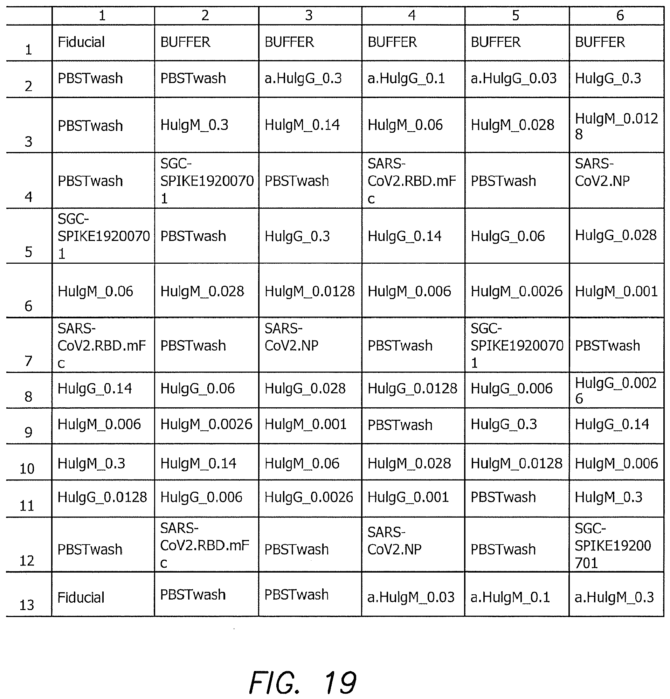

Fig. 19 is a diagrammatic depiction of the microarray of the embodiment for testing for Covid-19.

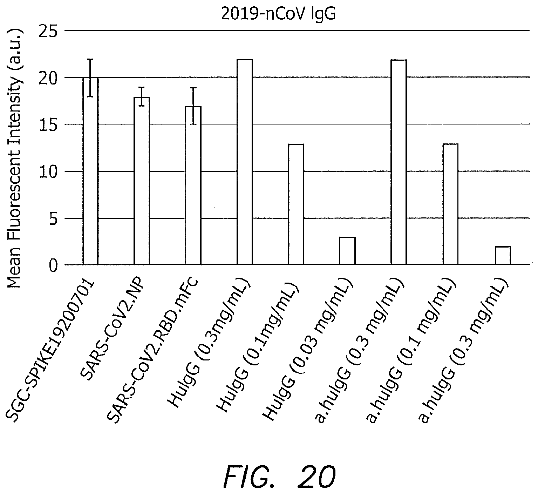

Fig. 20 is a bar graph which reports the value of each control spot, and mean value and standard deviation for each antigen.

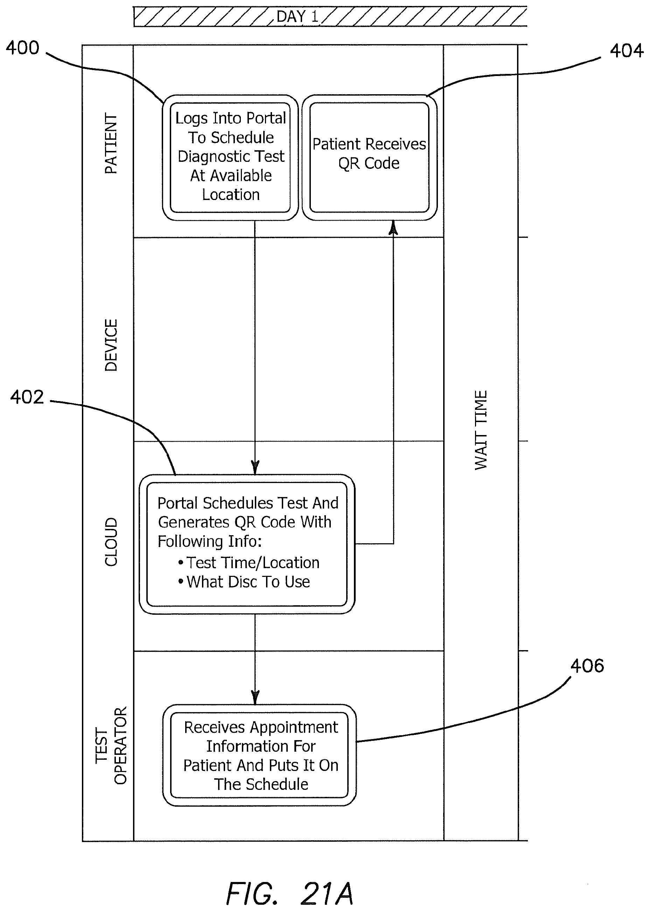

Figs. 21a- 21d is a diagram of the user flow or interaction with the system.

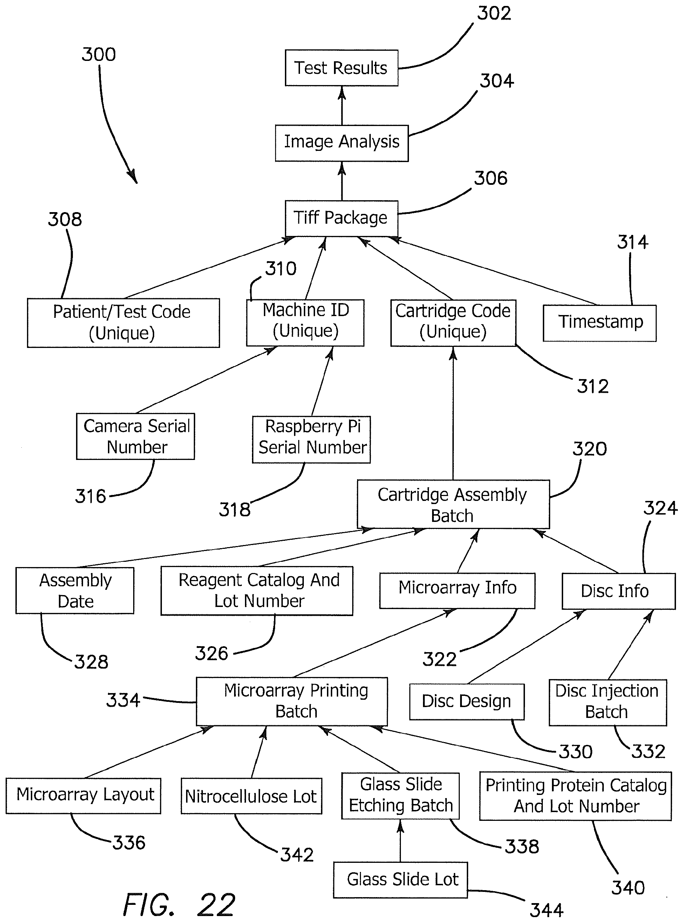

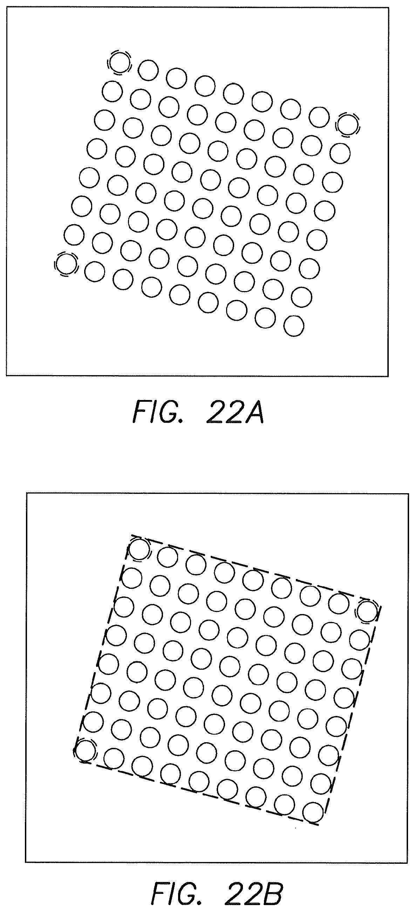

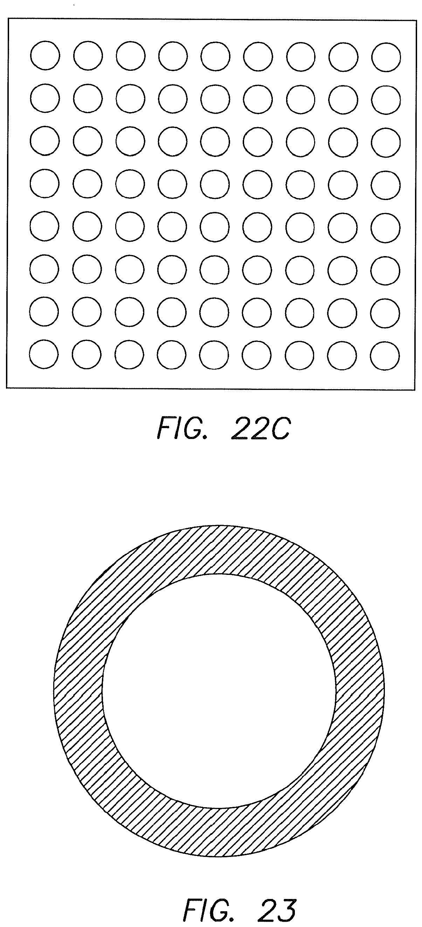

Fig. 22 is a tree graph of the data chain identification used to maintain data accountability for the tests and all involved components.

Fig. 22a is a diagram of an image of the microarray where after potential fiducials are identified, the program compares the distance ratios between all sets (combinations) of three contours, looking for ratios that match the theoretical fiducial spacing ratios given in the schema file identified in Fig. 22a as dashed circles.

Fig. 22b is a diagram of an image of the microarray where after determining the fiducials, a minimum fit rectangle is drawn around the three fiducials identified in Fig. 22b as a dashed rectangle.

Fig. 22c is a diagram of an image of the microarray where the minimum fit rectangle is then cropped and rotated so that the fiducials are located in the top left, bottom left, and top right as depicted in Fig. 22c.

Fig. 23 is a diagram of an image of a spot of the microarray the foreground median intensity is calculated and subtracted from the background mean intensity. Each spot is individually masked, and the median of each spot is calculated. Likewise, the mean of each background annulus is calculated and subtracted from the spot median as depicted in Fig. 23.

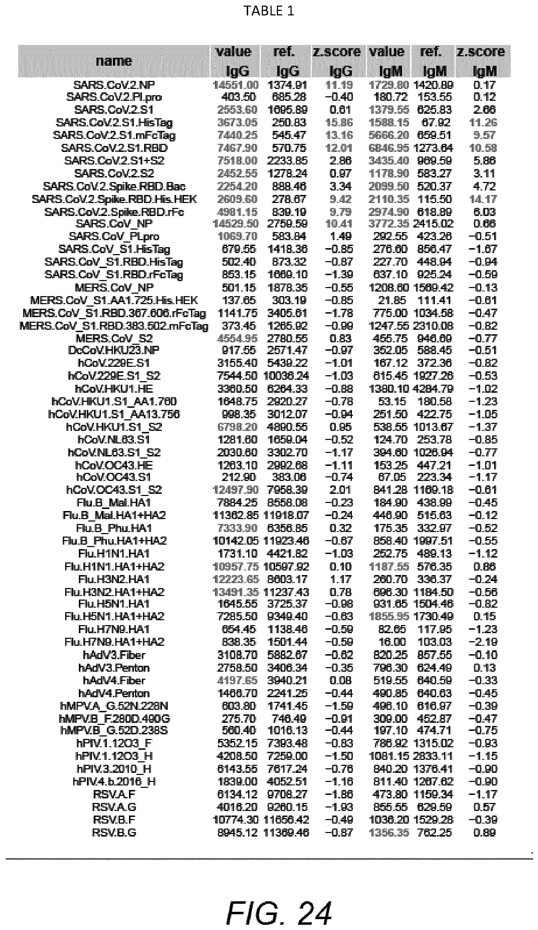

Figs. 24-25 illustrate tables 1 and 2, respectively.

The disclosure and its various embodiments can now be better understood by turning to the following detailed description of the preferred embodiments which are presented as illustrated examples of the embodiments defined in the claims. It is expressly understood that the embodiments as defined by the claims may be broader than the illustrated embodiments described below.

The apparatus of the illustrated embodiments include a backbone unit which includes the electronics, camera, optics, digital data gathering and communication via the internet to Cloud-based expert diagnostic servers, and electromechanical elements needed to provide field portable diagnostic testing of Covid-19 and other viral or bacterial infections. The same backbone unit supports at least three different microfluidic compact disks 68 (CDs) used for diagnostic assays or testing, namely for virology detection using a surface acoustical wave (SAW) detector, for microarray serology detectors for antibodies like IgG and IgM, and RT-PCR assays for nucleic acid targets using fluorescence detectors, which are denoted by Autonomous Medical Devices Inc. as its A10, A20 and A30 CD's respectively. The unit and its corresponding CDs are measurement or assay devices and do not perform high level diagnosis analysis, but provide the data needed to do so to fully developed diagnostic databases and expert systems resident in the Cloud in internet communication with the backbone unit.

*The Backbone Unit*

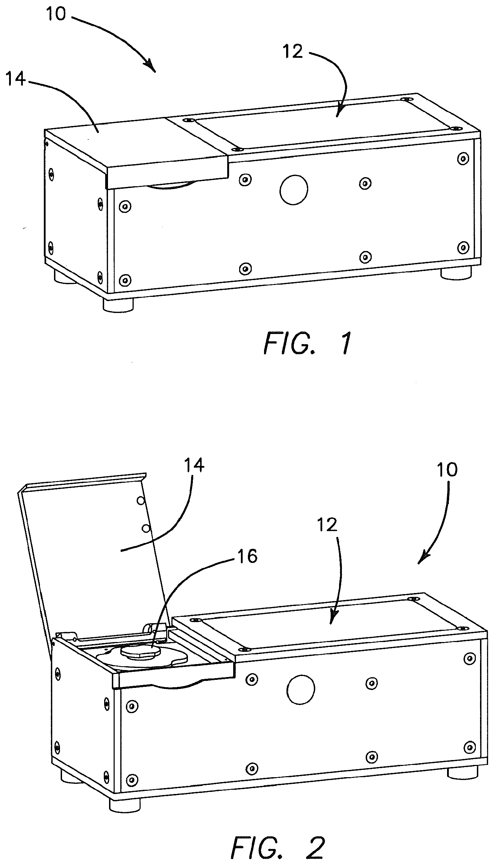

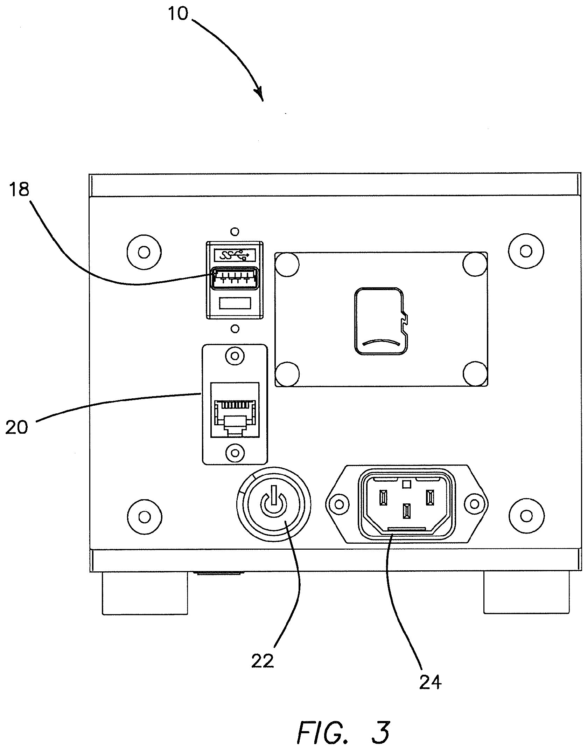

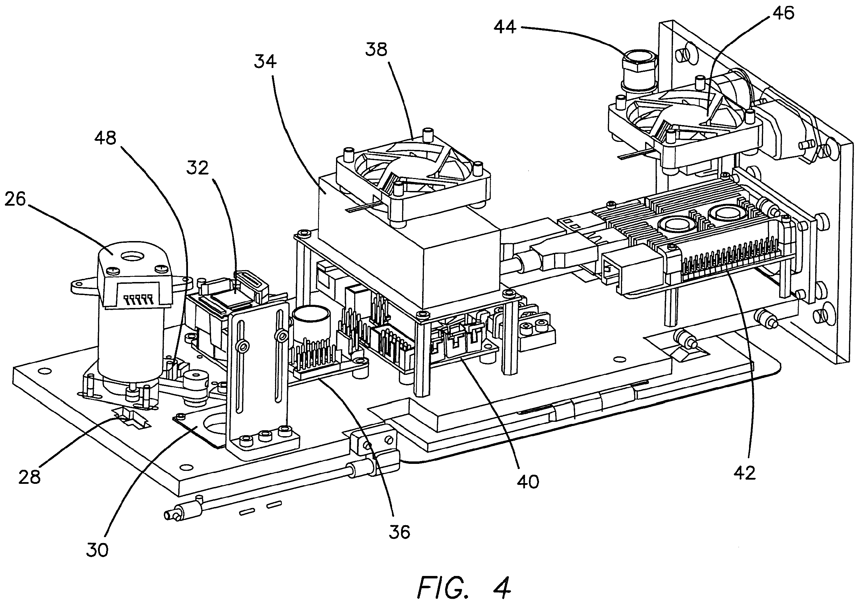

The backbone unit 10 shown in Fig. 1 is a desktop rectangular chassis with a color touch screen 12 on its top surface with a closeable lid 14 under which the microfluidic compact disks 68 (CDs) are placed on a spindle 16 shown in Fig. 2 for operation. More will be described below about the corresponding microfluidic disks 68. As shown in Fig. 3 one end of unit 10 is provided with a plurality of data and power connectors, such as external AC power receptacle 24, power switch 22, external USB port 20 and external Ethernet port 18. The primary electrical circuits, digital circuits, photonic elements, and electromechanical elements are depicted in perspective view of Fig. 4 which shows the interior layout of unit 10. Included among the pictured elements are CD motor 26 which spins the CDs, a CD index 28, a camera illumination subsystem 30, a camera 32, a power supply 34 with cooling fan 38, a motor controller 36 and a control board 40. To the side of motor 26 is a laser 48 used in CD operations, e.g. for opening selected valves in the CD. Also included is a CPU or Raspberry Pi 42, an electrical fuse 44 and a second cooling fan 46. On one of the long sides of unit 10, a quick response (QR) scanner (not shown) is also provided wherein patient information is integrated into the data output.

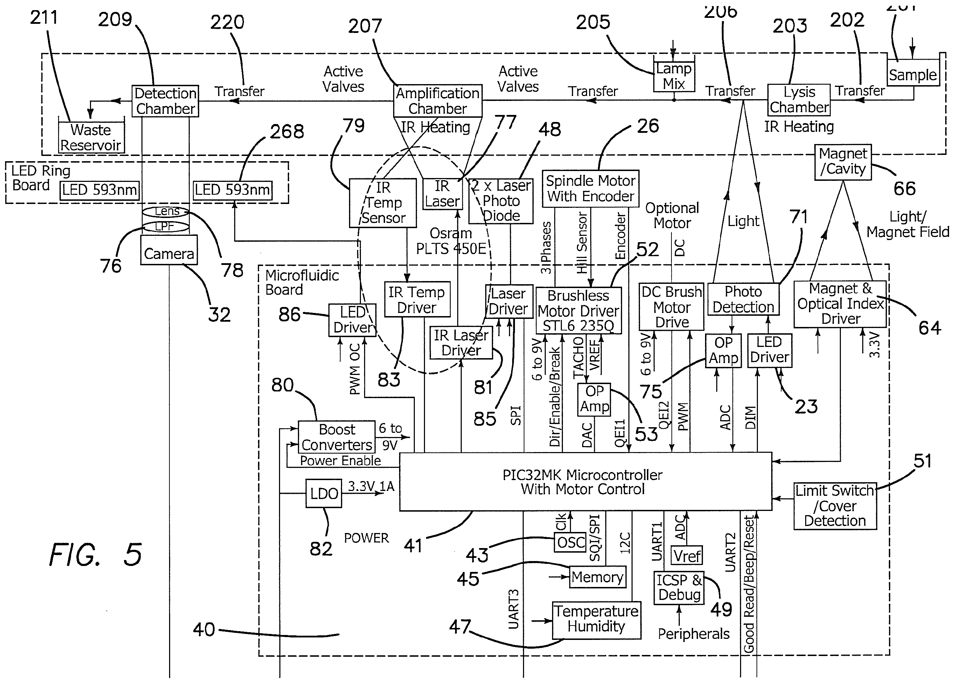

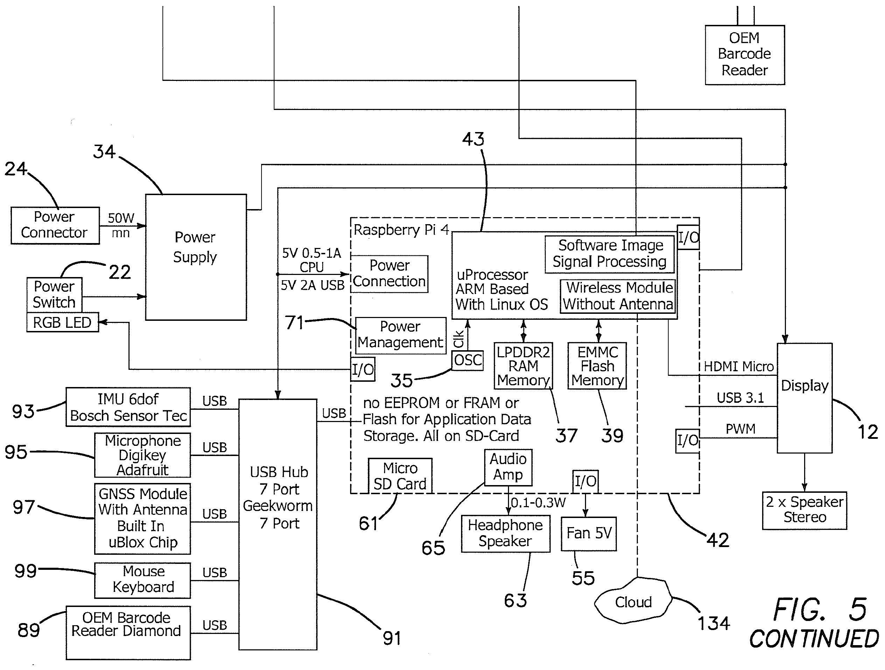

The operation of unit 10 is now better understood by referring to the block diagram of Fig. 5 which shows the supporting circuitry and photonics. A photonics control board 40, a CPU board 42 which is supported and coupled to power supply 34, provides a plurality of DC voltages (e.g. 5 and 24 VDC) and ground connections. CPU board 42 carries a raspberry pi 43 CPU, which is the main control circuit for unit 10 and handles all high-level programming commands, communications, and data handling. CPU 41 on photonics board 40 is a state machine and provides the needed drive and command signals to motor 26 and various LEDs 56 and lasers 48. CPU 41 controls the speed and rotation provided by motor 26 through direction-enable-break motor commands communicated to translated to brushless motor driver 52. Driver 52 also communicates a tachometer signal, TACHO to CPU 41, and receives a reference signal, VREF, from OpAMP 53 which in turn communicates to CPU 41 through an onboard digital-to-analog converter.

CPU 43 is an ARM-based (Advanced RISC machine) processor with a Linux operating system. CPU 43 is coupled to and drives camera 32 and provides raw image processing though a USB link to generate a transmissible digital data image through a wireless module ultimately to Cloud 134. CPU 43 is associated with a fan 55, clock 35, RAM memory 37 and an eMMC (embedded multimedia controller) flash memory 39, a micro-secure digital memory card (SD) 61, an audio amplifier 65 with headphone speakers 63, power management circuit 71 and a power connector 69. Memory card 61 is used to capture copies of the test results that are additionally transmitted on the cloud 134. The audio amplifier 65 is to be used with the speaker 63 which will transmit the health or status of the device to the user (test status, errors, etc). CPU 43 is coupled to display 12, both through HDMI and USB connections. Display 12 optionally drives a pair of stereo speakers 13 for communication to the user. CPU 43 is optionally communicated through a seven port USB hub 91 with a 6-degrees of freedom inertial measurement unit (IMU) 93, microphone 95, global navigation satellite system (GNSS) 97 with antenna, mouse/keyboard 99, barcode reader 89 allowing for location tracking, handing history, and user interaction and developer programming in the field.

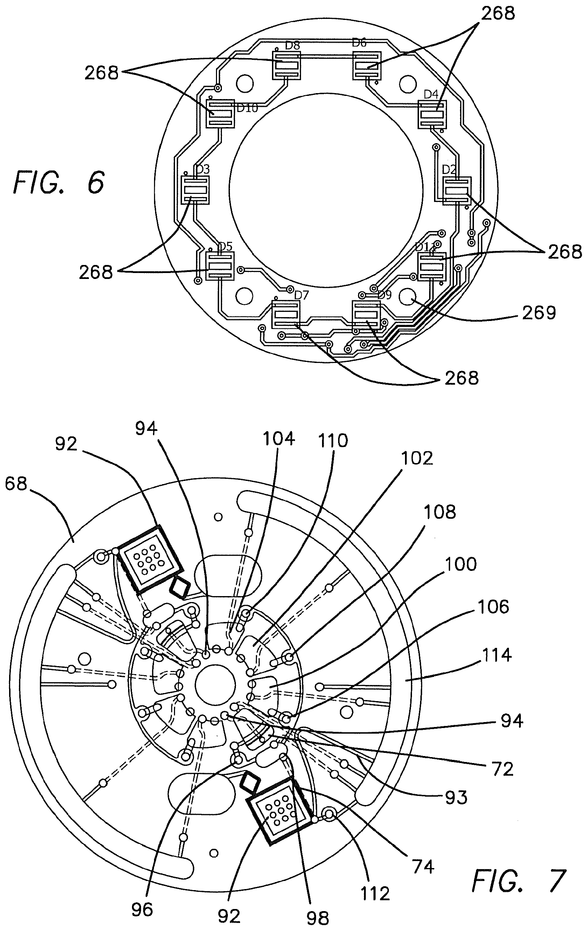

A microcontroller with CPU 41 with its memory 43 and external oscillator/clock 43 in photonics board 40 is coupled to CPU 42 and provides the controls for motor 26 according to the protocol shown in the flow diagram of Fig. 7 and various LEDs, lasers and sensors operationally associated with the RT- PCR process performed on disk 68. Using an on-chip digital-to-analog converter CPU 41 is coupled through an operational amplifier 53 to the reference and tachometer input/outputs of driver 52 as well as directly providing directional, enable and break motor commands to driver 52. CPU 41 commands brushless motor driver 52 to drive spindle or CD motor 26. Motor 26 includes an encoder whose signal is feedback to CPU 41 so that its speed, and direction of rotation is controlled in a closed loop servo mode. Driver 52 supplies 3-phase driving signals to motor 26, which includes a Hall Effect sensor returning an rpm feedback signal to buffer 52 indicative of the motor rpm. CPU 41 is also coupled to an encoder feedback signal from motor 26.

As shown in Fig. 5 photonic control board 40 is coupled to power supply 34 and includes a voltage boost circuit 80 increasing the 5V supply to 6V and a low dropout regulator (LDO) 82 (3.3V, 1A). CPU 41 is clocked by oscillator 43, and includes memory 45, a temperature/humidity sensor 47, an in circuit serial programming (ICSP) and debug interface 49, a source of a reference voltage VREF coupled to CPU 41 through an onboard analog-to-digital converter and a limit switch 51 built into unit 10's lid so that the motor 26 and all other photonics is shut down whenever the lid is lifted.

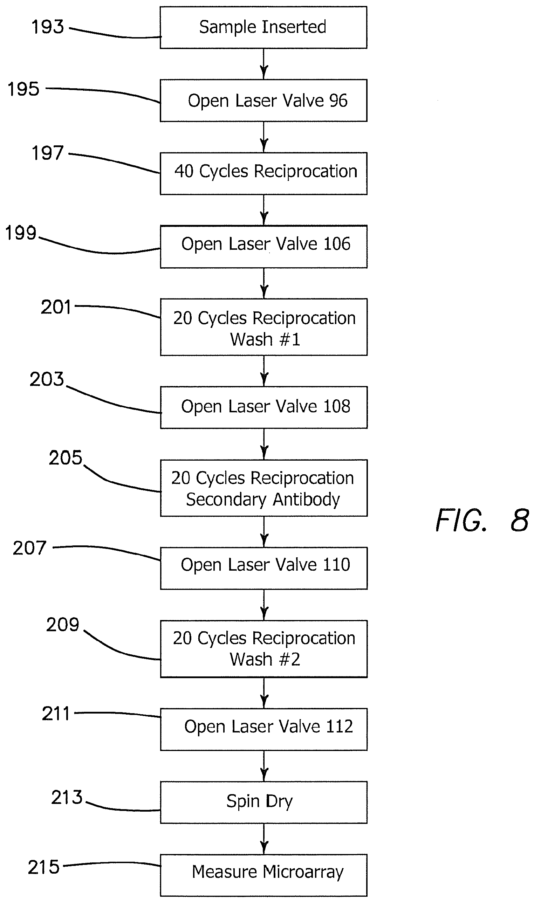

The operation of photonics board 40 with respect to disk 68 can now be understood. The movement and position of disk 68 is tracked by a disk mounted magnet 66 sensed by magnetic and optical index driver 64 coupled to CPU 41 by which the angular orientation or position of disk 68 is determined. The test sample is disposed into sample inlet 94 of Fig. 7 in step 93 of Fig. 8. The treated sample is transferred at step 95 to a blood-plasma separation chamber 72, served by sample inlet 94. After separation as described below in connection with Figs. 7 and 8, the separated plasma is transferred to receiving chamber 98 and then to microarray chamber 74 in which microarray 92 is disposed, where it is activated by 593nm LEDs 268 on an LED ring board 269 shown in Fig. 6, which are driven by LED driver 86 coupled to CPU 41. In the embodiment of Figs. 5 and 6, 10 LEDs, each operating at 593nm are providing in a ring surrounding the detection chamber 209 to provide a field of substantially even IR illumination to activate the fluorescent readout. Camera 32 takes a digital image of the fluorescently tagged sample through low pass filter 76 and lens 78, which image is communicated to CPU 42 from which it is transmitted to Cloud 134. The prepared sample can then be disposed in waste chamber 114. The bio-readout is the data the camera 32 captures fluoroscopically activated microarray 92. The fluorescence intensity corresponds to the concentration of the sample. The camera 32 detects the fluorescence of the microarray 92. Camera 32 is focused on the microarray chamber 72 through lens 76 and a low pass filter 78 for fluorescence imaging. An LED driver 86 is included in photonic controller 40 which drives the 593nm LEDs to activate the fluorescence of the tags in chamber 74.

*A20 - Disk Operation*

Before discussing diagnostic methods for Covid-19 on a microarray, turn now and consider the general operation of disk 68 when a microarray detector 92 is employed as depicted in the top plan view of Fig. 7. The elements described below on disk 68, which has a diameter of 70 mm and 4.5 mm thickness, are provided in duplicate to allow either redundant measurements to be made or two separate antibody tests using different microarrays 92 to be run simultaneously on the same patient. Disk 68 is made of clear plastic and has multiple chambers, channels and valves numerically machined therein as described in detail in the following. Disk 68 may be sealed on its top and bottom surfaces by a thin laminate layer of plastic. The method begins at step 193 with insertion of the sample taken from the patient at the point-of-care into the sample inlet 94. Disk 68 is spun at 5500rpm for 1 min at step 193 as depicted in the flow diagram of Fig. 8 to drive the sample into a blood-plasma separation chamber 72 where the centrifuging action separates the heavier blood constituents from the plasma. A first laser valve 62 is opened by positioning disk 68 so that laser valve 96, which is a meltable plug, is aligned with an underlying laser 48 in unit 10. The laser 48 is fired and valve 96 is opened and in about 0.5 min the plasma or serum flows from separation chamber 72 through receiving chamber 98 to microarray chamber 74 wherein microarray 92 is disposed. The transferred serum is reciprocated in microarray chamber 74 to react with the antibody dots of microarray 92 for about 5 min at step 197 for 40 cycles at 2700-5428 rpm followed by priming chamber 74 at 170rpm and then evacuating chamber 74 by rotation at 1000rpm through the primed siphon 93 into waste chamber 114.

At step 199, laser valve 106 is aligned with a laser 48 in unit 10 and opened with a 0.5min exposure. Thereafter, a wash buffer #1 stored in chamber 100 is transferred to microarray chamber 74 by reciprocation at step 201 for about 5 min at step 197 for 20 cycles at 2700-5428 rpm followed by priming chamber 100 at 170rpm and then evacuating chamber 74 by rotation at 1000rpm for about 2min.

At step 203, laser valve 108 is aligned with a laser 48 in unit 10 and opened with a 0.5min exposure. A secondary antibody stored in chamber 102 is transferred to microarray chamber 74 by reciprocation for about 5 min for 20 cycles at 2700-5428 rpm followed by priming chamber 102 at 170rpm and then evacuating chamber 74 by rotation at 1000rpm at step 205 for about 2 min. The secondary antibody is an anti-antibody. The antibody in blood binds to the antigen. The secondary antibody is an antibody that specifically binds to the tail of the antibody in the blood sample. This secondary antibody carries the fluorescent tag.

At step 207, laser valve 110 is aligned with a laser 48 in unit 10 and opened with a 0.5min exposure. Thereafter, a wash buffer #2 stored in chamber 104 is transferred to microarray chamber 74 by reciprocation at step 209 for about 5 min at step 197 for 20 cycles at 2700-5428 rpm followed by priming chamber 104 at 170rpm and then evacuating chamber 74 by rotation at 1000rpm for about 2min.

At step 211, valve 112 is aligned with a laser 48 in unit 10 and opened with a 0.5min exposure. At step 213, disk 68 is spun at 5500rpm for about 1 min to spin dry chamber 74 with wash #2 being evacuated to waste chamber 114. Chamber 74 and microarray 92 are then moved to align with camera 32 in unit 10. One or more grayscale images using induced fluorescence are taken by camera 32, stored and transmitted at step 215 in about 1 min by CPU 42 to the Cloud for data processing and diagnostic analysis as described below.

The total time needed to run the assay is about 16.5 minutes.

*Cloud Processing and Diagnosis*

Unit 10 performs the physical assay test using disk 68 and the detector provided in disk 68. What results in raw data in some form. Unit 10 does not further process the data nor analyze it to derive a diagnosis of the patient, but transmits the raw data to the Cloud, where remote servers provide processing and diagnostic analysis of the data. Using information associated with or in the patient's scanned QR code, the test results are then stored in a database and transmitted back to the patient's computer, smartphone or other electronic address of a health provider associated with the patient without further involvement with unit 10.

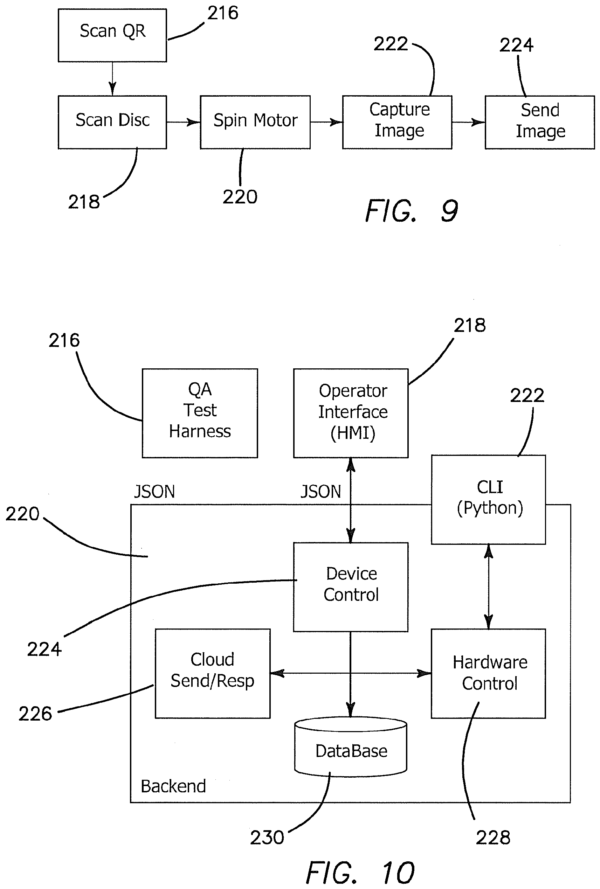

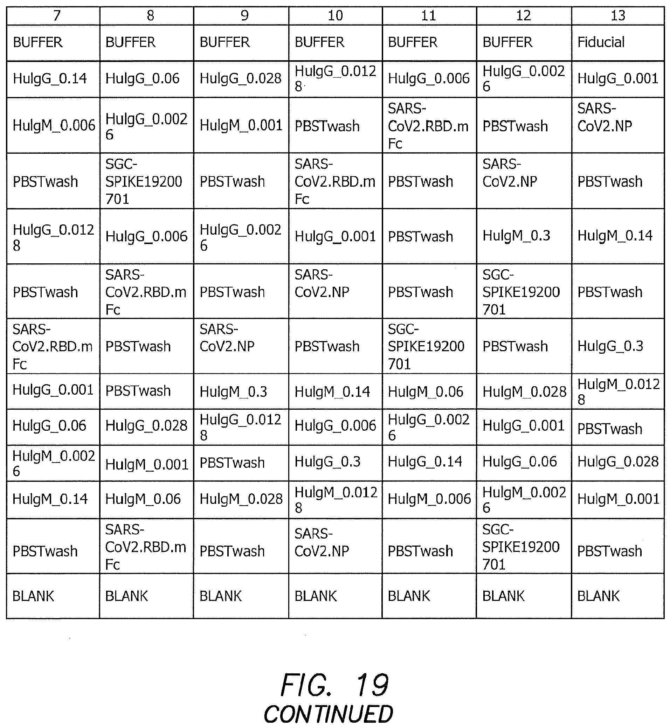

Fig. 9 is a diagram of data processing in the A20 at a high level. The patient's QR scan assigned to him or her by the health providers is scanned at step 216 associating a person with a test and at step 218 the disk barcode is scanned to associate a disk with the same test. The assay is run at step 220 as described above in connection with Figs. 7 and 8 ending with a captured fluorescently stimulated image of microarray 92 at step 222. Unit 10 then sends it captured grayscale image or images to the Cloud at step 224. At this point, unit 10's role in the test is ended.

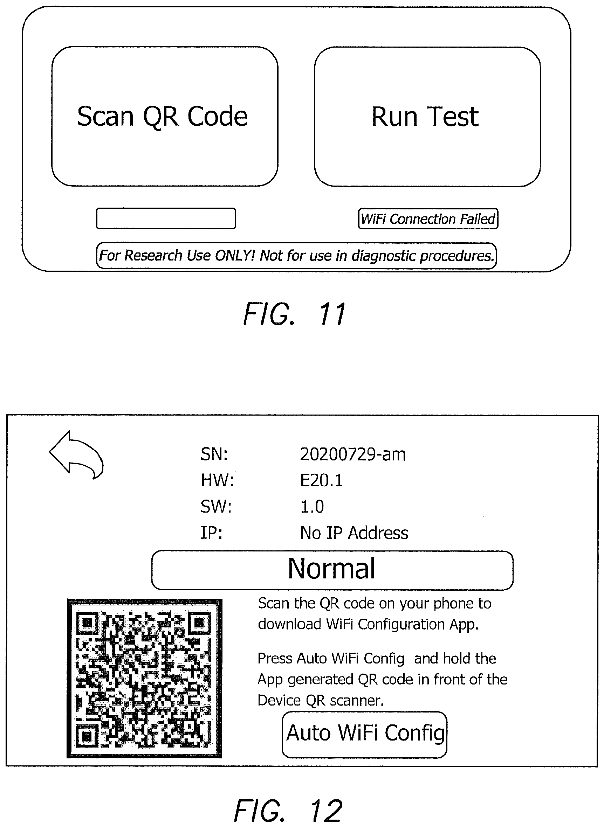

Prior to transmission of the captured data, unit 10 operates under software control as depicted in Fig. 10. A quality assurance test of the harness or wiring assemblies of unit 10 can be initiated by activating a QA Test Harness button 116 on touchscreen display 12 or a menu for operator interface (human- machine interface HMI) 118 activated, both of which operate with Java Script Object Notation (JSON). JSON is a type of data file that contains a human readable element. JSON is used because it is operating system agnostic, secure, and lossless (no data loss from the original data that comes off the camera sensor). Fig. 11 depicts a screenshot of touchscreen 12 when operator interface 118 is activated by a power on switch activation to display a Scan QR Code button and a Run Test button to scan the patient's QR code to associate the patient with the test and then to run the assay as described above respectively. Upon activation of the Scan QR Code button the operator then sees the screen of Fig. 12 and has access to the gear icon to allow setting the WiFI via a QR code. The gear icon is an icon on the human interface (GUI) which when touched, allows the user to enter the WIFI information for the device either manually or via a QR code.

Unit 10 operates autonomously under client/Python module 122, which includes responsive action to exterior communications as well as operating according to the onboard stored Linux Oracle programming protocol. The operator interface 118 communicates with the autonomously running backend software 120, which controls all operations of unit 10 through device control module 124. Major functions include Cloud bidirectional communication by Cloud module 126 hardware control module 128 and database module 130.

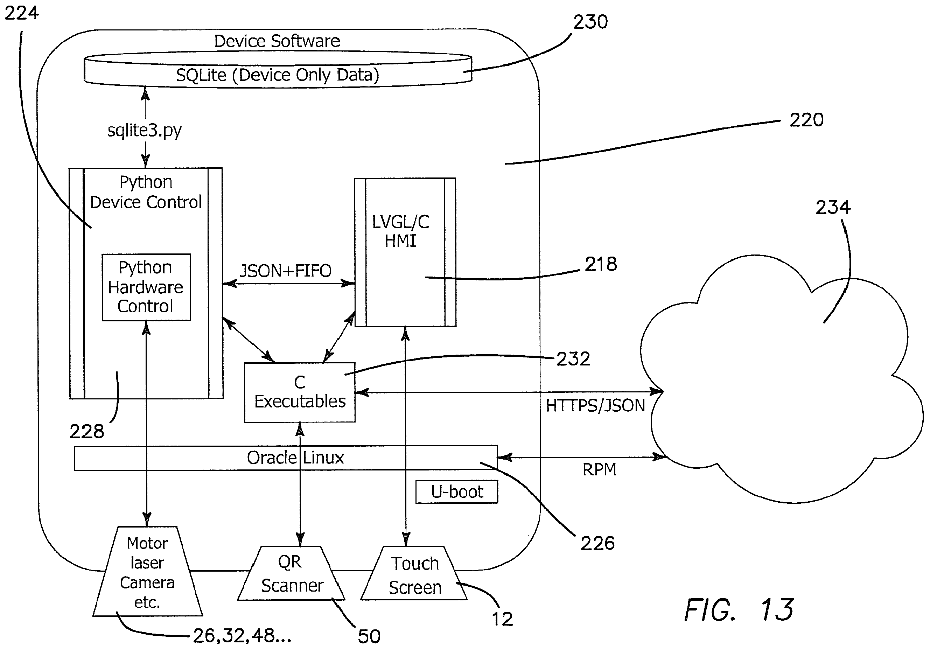

Fig. 13 illustrated the backend operation architecture. Database 130 is a SQLite device database module. SQLite is a widely used C-language library that implements a small, fast, self-contained, high-reliability, full-featured, SQL database engine. SQLite is an embedded SQL database engine. Unlike most other SQL databases, SQLite does not have a separate server process. SQLite reads and writes directly to ordinary disk files. A complete SQL database with multiple tables, indices, triggers, and views, is contained in a single disk file. SQLite is a compact library. Python device control 124 bi-directionally communicates with database module 130 and includes as an operating submodule Python hardware control 128, which controls CD motor 26, camera 32, lasers 48 and the other electronic and electromechanical devices of unit 10. Device control 124 is communicated via JSON and first-in-first-out (FIFO) with light and versatile graphics library (LVGL/C - HMI) 118, which is an open-source graphics library providing the tools needed to create an embedded graphic user interface (GUI) with graphical elements, visual effects and a low memory footprint made available to touchscreen 12. Both device control 124 and LVGL/C - HMI 118 are supported by a library of C executables library 132, which bi-directionally communicates with QR reader 50. Oracle or Linux based Cloud communication through module 126 to Cloud 134 with a red hat package manager (RPM) protocol which is used to store installation packages on Linux operating systems. C executables library 132 communicates with the Cloud 134 using a hypertext transfer protocol secure (HTTPS) encryption of JSON code.

*Image Processing in the Cloud*

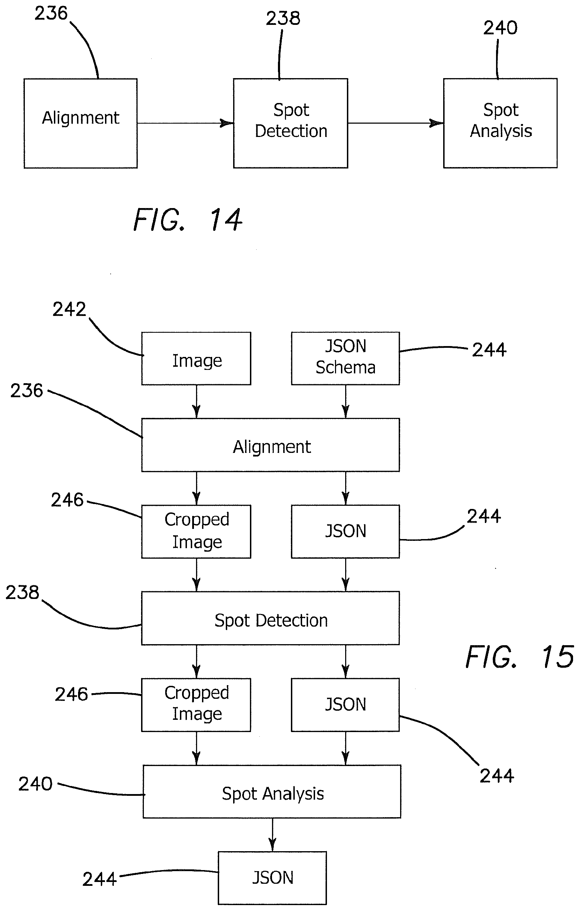

As described above unit 10 generates raw digital images taken by camera 32 and transmits them unprocessed to Cloud 134. The object is to convert the scanned microarray images into a scalar value for each microarray dot or site. The image data processing proceeds by the steps of alignment 136, spot detection 138 and spot analysis 140 as depicted in Fig. 14. Microarray spot image analysis is described in detail in Bell et.al. "An Integrated Digital Imaging System and Microarray Mapping Software for Rapid Multiplexed Quantitation of Protein Microarray Immunoassays," Grace Bio Labs, Bend, Oregon. The program structure of Fig. 15 has been written to keep each phase of the analysis modular. Each phase is passed an image 142, and a JSON information file 144. Each phase performs its work, and hands off the results for downstream processing.

The primary goal of the alignment step 236 is to correct for image inconsistencies, including angle of rotation, scale, and background noise. The alignment algorithm filters through all the shapes in an image, looking for objects that would qualify for spots or fiducials. After finding any potential spot or fiducial, the program looks for spacing ratios between all the potential fiducials that match the fiducial pattern indicated in the JSON schema file. Once the fiducials have been found, the image is rotated and cropped at step 246 to include only the region of interest. All processing is done on grayscale images.

Original or raw grayscale images 142 are imported into the program. The image 142 contains background information or noise that is not relevant to the processing of the image 142. The alignment phase aims to remove this region of noninterest (nROI) information by identifying the three bright fiducial spots at the corners of the microarray. A bilateral filter is applied to the image to reduce noise, but to keep sharp edges for downstream processing. Next, the image 142 is processed through an adaptive threshold filter to obtain a binary image of contours. Each contour is then filtered for a range of sizes or pixel areas. The size ranges are known beforehand and scale with the dimension of the image. Contours that are too large or too small are ignored. The remaining contours have a minimum fit circle drawn around their perimeter; the area of this circle is compared to the area of the contour to determine how 'circular' the contour is. Contours that have an area similar to the area of the bounding circle are retained. After potential fiducials are identified, the program compares the distance ratios between all sets (combinations) of three contours, looking for ratios that match the theoretical fiducial spacing ratios given in the schema file (Fig. 22a, dashed circles). The matching set of three contours are defined as the fiducials. After determining the fiducials, a minimum fit rectangle is drawn around the three fiducials (Fig. 22b, dashed rectangle). The minimum fit rectangle is then cropped and rotated so that the fiducials are located in the top left, bottom left, and top right (Fig. 22c). The location of each fiducial, and general information about the alignment routine is added to the JSON schema returned with the cropped image to be used by the next downstream application.

In the spot detection step 238 the primary purpose is to determine where each microarray spot is located within the region of interest image. This will be used downstream to determine each spot value. Using the fiducial locations and known size of the microarray, the cropped image is subdivided into a grid, where each square should contain a spot. Adaptive thresholding is applied within each square of the grid. The adaptive threshold image of each square is used to calculate the image moment, which is used to determine centroids for spots:

$$C = \frac{\sum_{p=1}^{N} I_p \left( \overline{X_p} + \overline{Y_p} \right)}{\sum_{p=1}^{N} I_p}$$

Where $I_p$ is the pixel intensity at the pixel $p$, $\overline{X_p}$ and $\overline{Y_p}$ are the distance vectors to the pixel $p$ relative to a reference point, and $N$ is the total number of pixels in the grid region. Spot diameters are measured in each square if detected. If no visible spot is detected, each spot is assigned the average diameter of found spots.

The purpose of the spot analysis phase is to assign a single scalar value to each spot in the grid. Currently this is done by calculating the foreground median intensity and subtracting it from the background mean intensity. Each spot is individually masked, and the median of each spot is calculated. Likewise, the mean of each background annulus is calculated and subtracted from the spot median (Fig. 23). The analysis output value is packed into the JSON structure for each spot and returned as a final result.

*Diagnostic Processing the Cloud*

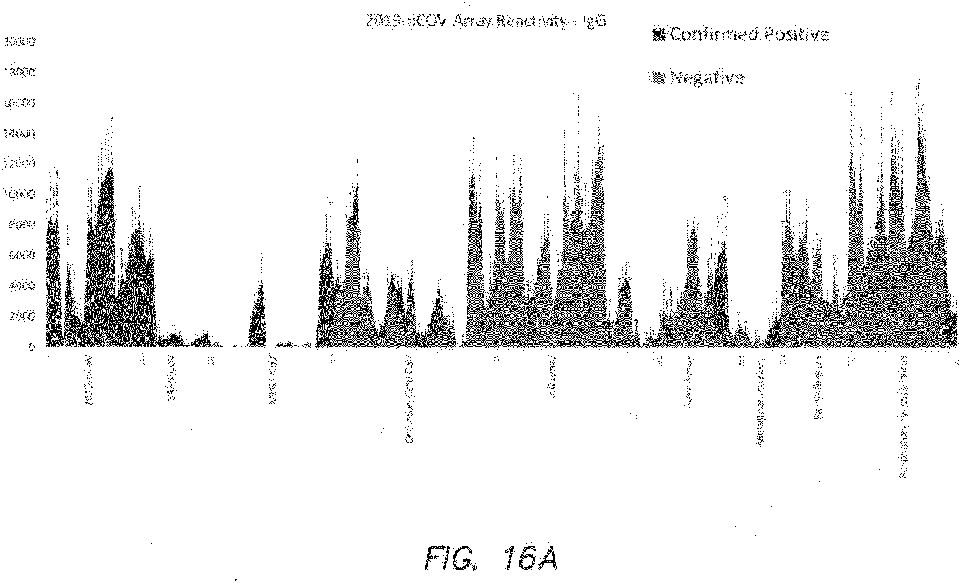

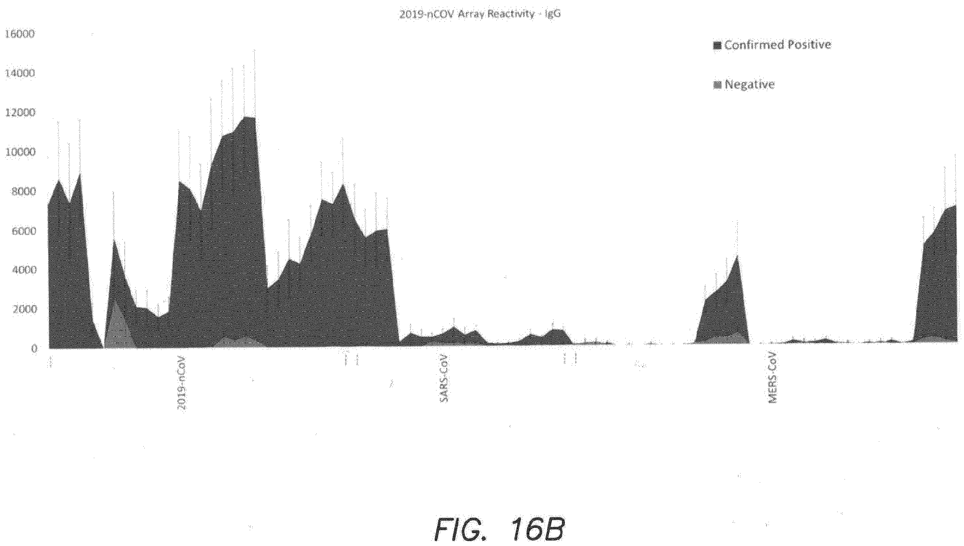

Before considering the details of diagnostic processing of the processed image data in the Cloud, turn first and consider the microarrays used in the illustrated embodiments. The "multiplexed antibody array" in disk 68 provides an individual's virus "exposure fingerprint", the "legacy antibody profile" reflecting past exposure and vaccination history. This array analysis approach is significantly more data rich (e.g. 67 antigens with 4 replicates per array) and is more quantitative than lateral flow assays in current use for measuring antibodies against the virus. To appreciate this point turn to Figs. 16a and 16b where both positive and negative 2019 nCOV Array Sensitivity IgG results are shown obtained on blood samples from the COVID-19 Washington State 2020 outbreak.

High throughput cloning and constructing microarrays have previously been developed that contain human and animal antibodies with antigens from more than 35 medically important pathogens, including bacteria, parasites, fungi and viruses such as vaccinia, monkey pox, Herpes 1 & 2, Varicella zoster, HPV, HIV, Dengue, influenza, West Nile, Chikungunya, adenovirus, and coronaviruses. A DNA microarray (also commonly known as DNA chip or biochip) is a collection of microscopic DNA spots attached to a solid surface. DNA microarrays are used to measure the expression levels of large numbers of genes simultaneously or to genotype multiple regions of a genome. Each DNA spot contains picomoles (10-12 moles) of a specific DNA sequence, known as probes (or reporters or oligos). These can be a short section of a gene or other DNA element that are used to hybridize a cDNA or cRNA, also called anti-sense RNA, sample, called target, under high-stringency conditions. Probe-target hybridization is usually detected and quantified by detection of fluorophore-, silver-, or chemiluminescence-labeled targets to determine relative abundance of nucleic acid sequences in the target. The original nucleic acid arrays were macro arrays approximately 9 cm $\times$ 12 cm and the first computerized image-based analysis was published in 1981. Over 25000 samples were probed from humans and animals infected with pathogens and identified over 1000 immunodominant and candidate vaccine antigens against these pathogens. It has been shown that the individual proteins/antibodies printed on these arrays 92 capture antibodies and/or antigens present in serum from infected individuals and the amount of captured antibody can be quantified using fluorescent secondary antibody.

In this way a comprehensive profile of antibodies that result after infection or exposure can be determined that is characteristic of the type of infection and the stage of diseases. Arrays 92 can be produced and probed in large numbers (>500 serum or plasma specimens per day) while consuming <2$\mu$l of each sample. This microarray approach allows investigators to assess the antibody repertoire in large collections of samples not possible with other technologies.

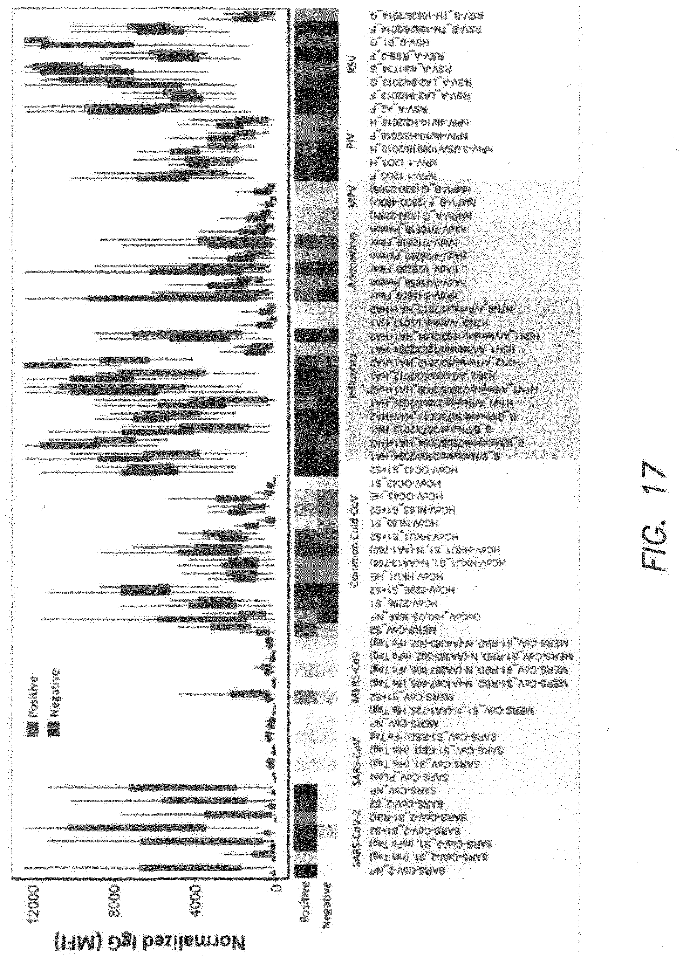

A coronavirus antigen microarray 92 (COVAM) was constructed containing 67 antigens that are causes of acute respiratory infections. The viral antigens printed on this array 92 are from epidemic coronaviruses including SARS-CoV-2, SARS-CoV, MERS-CoV, common cold coronaviruses (HKU1, OC43, NL63, 229E), and multiple subtypes of influenza, adenovirus, metapneumovirus, parainfluenza, and respiratory syncytial virus. The SARS- CoV-2 antigens on this array 92 include the spike protein (S), the receptor- binding (RBD), S1, and S2 domains, the whole protein (S1+S2), and the nucleocapsid protein (NP) as shown in the graph of Fig. 17. There is a similar set of antigens represented on the array from SARS-CoV, MERS-CoV, and the four common cold corona viruses.

To determine the antibody profile of SARS-CoV-2 infection, the differential reactivity to these antigens was evaluated for SARS-CoV-2 convalescent blood specimens from PCR-positive individuals (positive group) and sera collected prior to the COVID-19 pandemic from naive individuals (negative control group). As shown in the heatmaps of Figs. 17a and 17b, the positive group is highly reactive against SARS-CoV-2 antigens. This is more evident for the IgG than for IgA. The negative controls do not react to SARS- CoV-2, SARS-CoV or MERS-CoV antigens despite showing high reactivity to the common cold coronavirus antigens. Positive group displays high IgG reactivity to SARS-CoV-2 NP, S2, and S1+S2 antigens and to a lesser degree SARS-CoV-2 S1 shown in Figs. 17a and 17b. The positive group also demonstrates high IgG cross-reactivity against SARS-CoV NP, MERS-CoV S2 and S1+S2 antigens, while the negative group demonstrates low cross-reactivity with S1+S2 and S2 antigens from SARS-CoV-2 and MERS-CoV and no cross-reactivity against other SARS-CoV-2 antigens.

Table 1, shown in Fig. 24, contains the fluorescence intensity results for IgG shown in Fig. 18a, the Z-score statistics for the fluorescence results in Fig. 18c, the fluorescence intensity results for IgM shown in Fig. 18b, and the Z-score statistics for the fluorescence results of Fig. 18d. The Z-score shows how many standard deviations above (positive) or below (negative) the mean negative results a confirmed positive IgG or IgM sample is. Statistically significant Z- scores (5 or greater) have shaded numerals.

Antigens were then evaluated to discriminate the positive group from the negative group across a full range of assay cutoff values using receiver- operating-characteristic (ROC) curves for which an area-under curve (AUC) was measured. High-performing antigens for detection of IgG are defined by ROC AUC >0.85 as shown in Table 1. Four antigens are ranked as high-performing antigens: SARS-CoV-2 NP, SARS-CoV NP, SARS-CoV-2S1+S2, and SARS-CoV-2_S2. Additional high-performing antigens included SARS-CoV-2 S1 (with mouse Fc tag) and RBD, and MERS-CoV S2. The optimal sensitivity and specificity were also estimated for the seven high-performing antigens based on the Youden Index. Youden's J statistic (also called Youden's Index) is a single statistic that captures the performance of a dichotomous diagnostic test. Informedness is its generalization to the multiclass case and estimates the probability of an informed decision. The lowest sensitivity was seen for SARS- CoV-2 S1, which correlates with the relatively lower reactivity to this antigen in the positive group. The lowest specificity was seen for SARS-CoV-2 S2, which correlates with the cross-reactivity for this antigen seen in a subset of the negative group. To estimate the gain in performance by combining antigens, all possible combinations of up to four of the seven high-performing antigens were tested in silico for performance in discriminating the positive and negative groups. The ROC curve with AUC, sensitivity, and specificity was calculated for each combination. There is a clear gain in performance by combining two or three antigens. For IgG, the best discrimination was achieved with the two-antigen combination of SARS-CoV-2S2 and SARS-CoV NP, with similar performance upon the addition of SARS-CoV-2S1 with mouse Fc tag (AUC = 0.994, specificity = 1, sensitivity = 0.944). The addition of a fourth antigen decreased the performance.

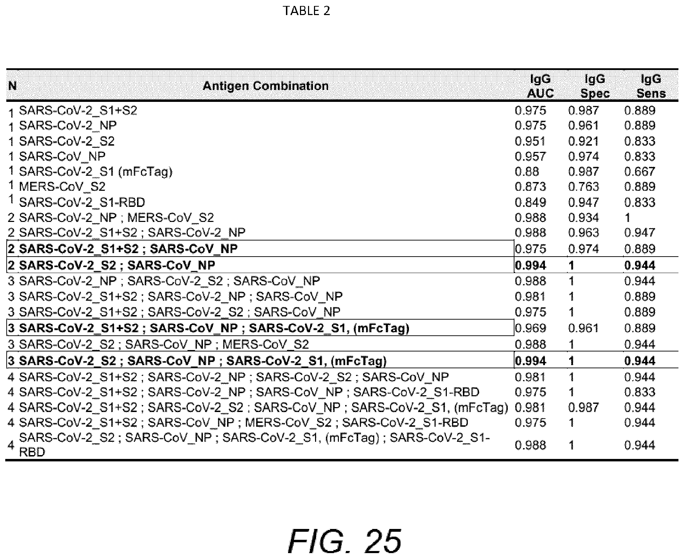

Table 2, shown in Fig. 25, shows the performance data for combinations of high-performing antigens. ROC, AUC values and sensitivity and specificity based on Youden index for discrimination of positive and negative sera were derived for each individual antigen ranked, and high-performing antigens with ROC AUC >0.86 are indicated above the lines.

Figs. 18a-d show an example of a single confirmed positive patient results. Fig. 18a shows the normalized fluorescence intensity for various IgG antibodies in a serum, with the two lines showing the average results for a confirmed positive (top) and confirmed negative (bottom). Fig. 18b shows the normalized fluorescence intensity for various IgM antibodies in a serum, with the two lines showing the average results for a confirmed positive (top) and confirmed negative (bottom). Fig. 18c shows the plotted Z-scores for the IgG antibodies between a positive and negative result, with the three dotted lines representing the various Z-score thresholds for mild, moderate, and significant response. Fig. 18d shows the plotted Z-scores for the IgM antibodies between a positive and negative result, with the three dotted lines representing the various Z-scores.

More particularly, the A20 serology test is an optical microarray test that performs an indirect immunofluorescence assay for qualitative detection of IgM and IgG antibodies to SARS-CoV-2 in human blood. The serology test is intended for use as an aid in identifying individuals with an adaptive immune response to SARS-CoV-2, indicating recent or prior infection. The serology test currently produces an image of the microarray and a graph of the intensities of the spots on the array. To develop a diagnostic standard known RT-PCR positive and negative samples are tested on the apparatus described above. This establishes cutoff thresholds for reactivity to each of the three SARS-CoV-2 antigens in the microarray, which enables the apparatus to autonomously provide a qualitative "yes" (reactive) or "no" (non-reactive) result.

*Microarray Description*

The serology test contains two identical microarrays on disk 68, one for testing IgG presence and the other for IgM presence. The two classes of antibodies are probed separately by using IgG or IgM reporter antibodies. Each of the two microarrays has the form diagrammatically depicted in Fig. 19. The microarray spots are characterized as:

a. Negative Controls: BUFFER (10 spots): Phosphate-buffered saline (PBS) with 0.001% Tween-20 (Polyethylene glycol sorbitan monolaurate, Polyoxyethylenesorbitan monolaurate). These spots are printing buffers and serve as a negative control to determine the baseline fluorescence of the array.

b. Positive Controls 1: HuIgG (5 spots): Human IgG printed in concentrations of eight dilutions from 0.3 to 0.001 mg/ml for a total of 40 spots. These spots serve as a positive control to indicate that the reporter antibody for IgG is performing appropriately to accurately determine cutoff values of the array when testing on serum samples. The concentration ladder can serve as a rough guide to interpret the microarray's fluorescence.

c. HuIgM (5 spots): Human IgM printed in concentrations of eight dilutions from 0.3 to 0.001 mg/ml for a total of 40 spots. These spots serve as a positive control to indicate that the reporter antibody for IgM is performing appropriately to accurately determine cutoff values of the array when testing on serum samples. The concentration ladder can serve as a rough guide to interpret the microarray's fluorescence.

d. Positive Controls 2: a.HuIgG (3 spots): anti-Human IgG printed in concentrations of 0.3, 0.1, and 0.03 mg/ml. These spots serve as a positive control to indicate that there are human IgG antibodies in the sample. a.HuIgM (3 spots): anti-Human IgM printed in concentrations of 0.3, 0.1, and 0.03 mg/ml. These spots serve as a positive control to indicate that there are human IgM antibodies in the sample.

e. Antigens: SGC-SPIKE19200701 (8 spots): SARS-Cov-2 Spike Protein (University of Oxford). Printed at 0.2 mg/ml. SARS-CoV2.NP (8 spots): SARS-Cov-2 Nucleocapsid Protein (Sinobiological). Printed at 0.2 mg/ml. SARS-CoV2.RBD.mFc (8 spots): SARS-Cov-2 Spike Protein (RBD, mFc Tag) (Sinobiological). Printed at 0.2 mg/ml.

f. Fiducial (3 spots): Streptavidin, Alexa Fluor 647 conjugate. These spots are designed to be the brightest spots on the array and are used to locate and orient the array.

g. PBSTwash (21 spots): PBS + 0.05% tween20 used for washing pins.

h. Blank (2 spots): Unused microarray locations.

*Microarray Results*

The images of each microarray in an A20 serology test are uploaded to a server on the Oracle Cloud for analysis. After the corner fiducials are used to locate and orient the microarrays, the images are analyzed to produce scalar values for each spot in the microarray. These measurements are the median fluorescence intensity of each spot, minus the mean fluorescence intensity of the surrounding annulus. These measurements will be available to the user online in a file in JSON format, along with a plot summarizing the values of the three SARS-CoV-2 antigens printed on the microarray. The JSON file is a hierarchical file with the following top-level structure:

*Top-Level Overview of JSON*

```
{
        "diskTypeID": "1234-02",
        "spots": [
                {...},
                {...}
        ],
        // information about the grid analysis
        "gridInfo": {
                "info": "Grid Detect",
                "version": "0.1",
                "avg_spot_dia": 83
        }
}
```

The measurements for each spot are contained in a list in the "spots" entry, with thorough details of each spot:

*JSON Details*

```
{
        // The QR code on the disk.
        "diskTypeID": "1234-02",
        // Array of all 'spots' on the microarray image
        "spots": [
                {
                        // spot row
                        "row": "2",
                        // spot col
                        "column": "5",
                        // group name if multiple virus-specific antigens are used
```

```
"group": "",
// name of the spot
"id": "SGC-SPIKE19200701",
// (x,y) pixel position of the spot
"position": [
    329,
    146
],
// Array of different types of analyses
"analysis": [
    {
        // Name of the analysis method
        "name": "Spot Mean - Donut Median",
        // Version of this analysis method
        "version": "0.0",
        // Final value of this analysis method
        "value": 1.2824578790882057
    }
]
},
// {...} many more spots //
]
}
```

The accompanying summary figure of each microarray is a bar chart, which reports the value of each control spot, and mean value and standard deviation for each antigen such as shown in an example in Fig. 20. From these results a conventional statistical model to distinguish blood samples with and without anti-SARS-CoV-2 antibodies is established.

*Overall System Usage*

The overall user flow or user interaction with the system is illustrated in Figs. 21a - 21e. In Fig. 21a the action of the patient, unit 10, Cloud 134 and the test operator running unit 10 are each identified in four horizontal rows. At step 400 the patient logs into a portal on the internet to schedule a diagnostic test at an available test location. The remote server in Cloud 134 communicated to the portal schedules the patient's test at step 402 and generates a unique QR code which has: 1) the test time and location; and 2) which test to run, i.e. whether a disk 68 associated with A10, A20 or A30 is to be run. The QR code is how the patient controls the use of the testing information and its privacy. The QR code is sent to the patient at step 404, which the patient downloads into his or her smartphone, laptop, or computer. Meanwhile at step 406 the remote server in Cloud 134 transmits the appointment information for the patient and inserts it into the testing schedule. At this point the procedure there may be a pause of one or more days before further action is taken.

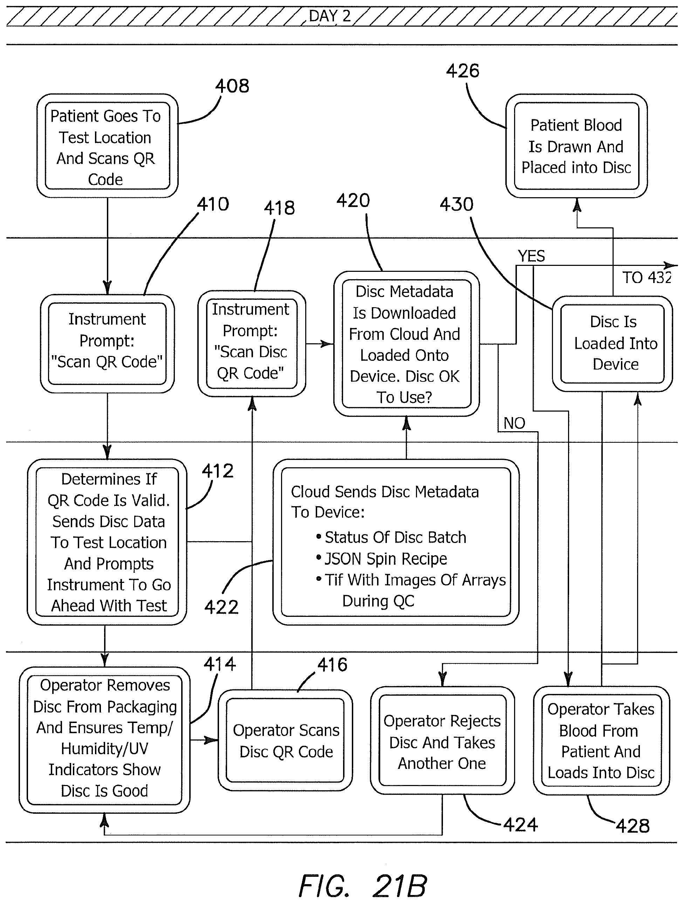

On the day of the appointment at step 408 in Fig. 21b the patient goes to the testing location and scan his or her QR code into unit 10 in response to a screen prompt at step 410. The validity of the QR code is checked at step 412 in Cloud 134 and the remote server communicates to the testing site which of the disks 68 is to be used for the test, authorizing unit 10 to use a specific type of disk 68. The test operator, after checking at step 414 the humidity and temperature levels on the disk packaging to verify the integrity of disk 68, scans the disk's QR code at step 416 in response to a screen prompt from unit 10 at step 418. Cloud 134 communicates the disk's metadata to unit 10 at step 422, which metadata includes the status of the disk batch, a JSON file for the spin protocol, and grayscale TIF files of the microarrays 92 generated during quality control testing for the disk 68. Unit 10 downloads the disk's metadata from Cloud 134 at step 420 and the authority to use disk 68 in the test is determined.

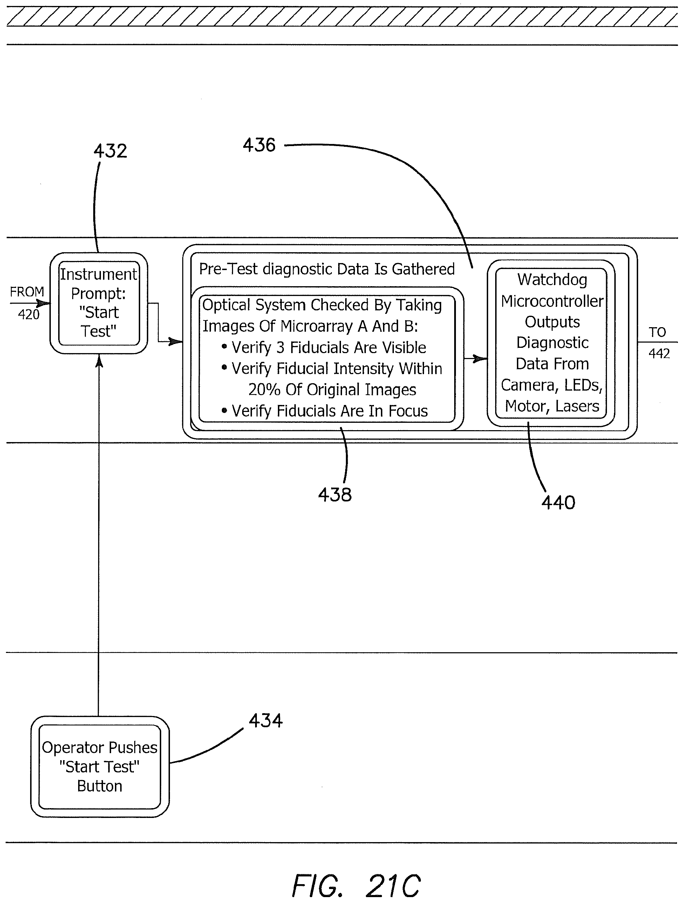

If it is determined at step 420 that the authority to use disk 68 is denied, the operator is advised to reject disk 68 and replace it with another at step 424, after which the procedure returns to step 414. If use of disk 68 is authorized, then a blood sample, such as a finger prick, is taken from the patient by the test operator at step 426, loaded by the test operator into disk 68 at step 428, and disk 68 then loaded into unit 10 at step 430. Unit 10 displays a screen prompt to the test operator to begin the test at step 432 in Fig. 21c. In response the test operator touches the start button on the screen display at step 434.

Pretest diagnostic data is gathered in step 436, this includes checking the optical system at step 438 with both microarrays 92 in disk 68 by verifying that: 1) the three fiducial spots in each array are visible; 2) the fiducial intensity is within 20% of the original images of the microarray; and 3) the fiducial spots are in focus. Similarly, a watchdog routine in COU 43 at step 440 outputs diagnostic data from camera 32, the LEDs 56, motor 26, and lasers 48. Thereafter, unit 10 runs a spin protocol on disk 68 at step 442 as described above and takes a grayscale image of each microarray 92 at the end of the assay. The watchdog routine in CPU 43 at step 444 continues to monitor unit 10 during the assay procedure and generates an error message display in the event of a fault and stops the test or assay if needed.

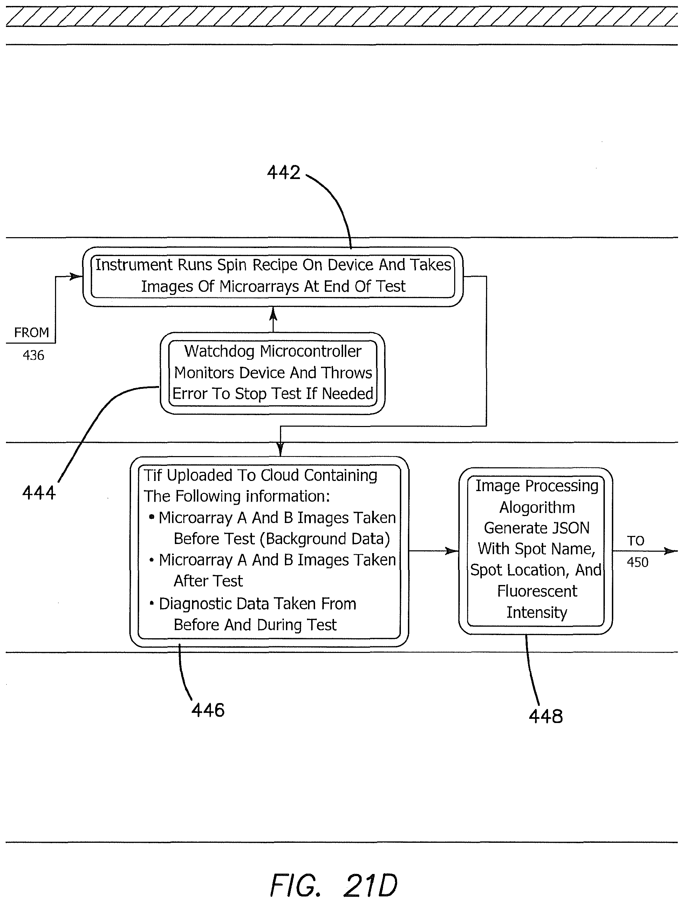

The grayscale TIF image taken by camera 32 of each microarray 92 is uploaded to Cloud 134 at step 446 in Fig. 21d both of each microarray before the test to provide background data, after the test to provide test data, and files including the diagnostic data taken before and after the test. At step 448 an image processing algorithm as described above processes the digital images of microarrays 92 to generate a JSON file listing each spot name, spot location and fluorescence intensity, from which a diagnosis is made.

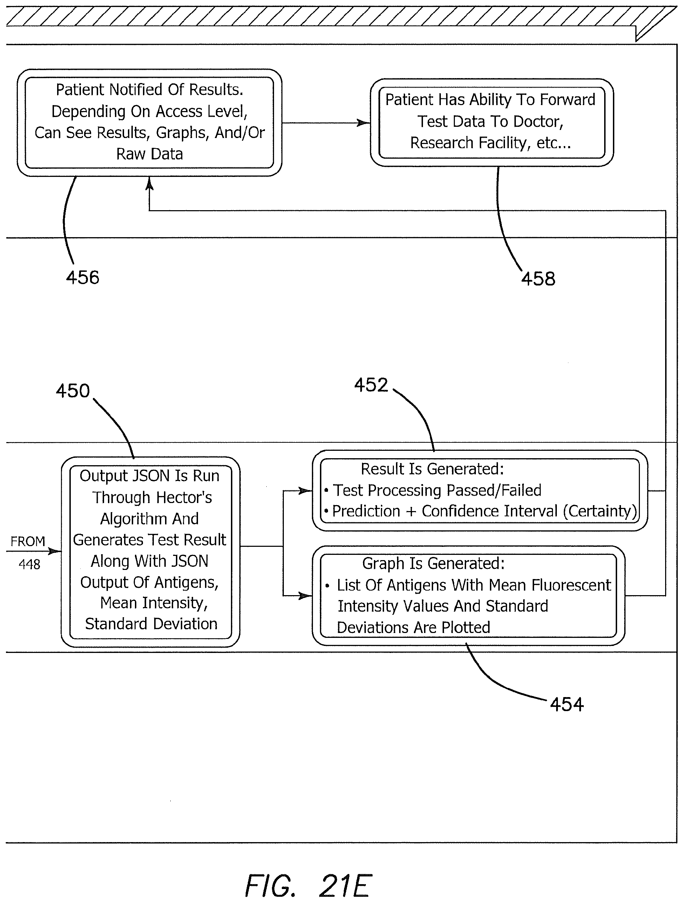

From the JSON output file the test processing is determined as being passed or failed at step 452 in Fig. 21e. If the test passed, a predictive diagnosis is made of the diagnosis and a confidence interval calculated. The predictive diagnosis is provided from a statistical model such as logistic regression or random forest, using fluorescence intensity or calculated Z-scores. At the same time at step 454 a list of antigens with mean fluorescent intensity values and standard deviations is plotted. These results are communicated from Cloud 134 to the patient's smartphone, laptop, or computer at step 456, and depending on the access level granted, the patient can see the results, graphs and/or raw data. At step 458 the patient then has the choice to forward the test data to his or her doctor, healthcare technician, research facility, governmental authority or wherever the patient deems necessary.

*Data Chain Identification*

Control of the data sent to the remote server in Cloud 134 is realized utilizing the identification chain 300 of Fig. 22. This identification chain 300 can be viewed as a tree graph as shown in Fig. 22, where each box is a node in the tree that can be traversed in either direction, and where each node corresponds to a manufactured component. Each node in the chain can be queried recursively to yield information of its corresponding components, or its own details of manufacture catalog number, date, origin, and batch. Each test 302 is encoded in a transmitted image analysis data file 304, which includes a TIFF package 306, which in turn is tied to a unique patient/test code 308, a unique machine ID 310, a unique cartridge code 312, and a UTC timestamp of the performed test. Connecting the test code to the patient/test code 308, machine ID 310, and timestamp 314 guarantees that no two test results can be misidentified, as two tests cannot be performed on the same machine at the same time.

Attaching a unique cartridge code 312 further guarantees the uniqueness of each test and its results, but also creates a complete identification chain to connect a particular test 302 and its results to every relevant assembly component involved in that test 302. This provides full traceability, allowing one to identify all component lot numbers used in a particular disc 68, or all discs 68 utilizing a particular component lot number. This allows one to acquire data from compromised tests and determine a faulty component lot or recall all discs that utilize a faulty component lot.

The machine ID 310 is uniquely defined by its camera serial number 316 and on-board computer (pi raspberry) serial number 318. The machine ID 310 can then provide the hierarchy of all sub-assemblies of all its mechanical and electrical components.

The cartridge code 312 is traced to the cartridge assembly batch 320, which details the date of assembly 328, microarray information 322, disc information 324, and reagent catalog and lot number 326 stored on the cartridge. The disc information 324 contains details of the disc design 330 and disc injection batch 332. The microarray information 322 contains details of the printing date 334, the microarray layout 336, the glass slide etching batch 338, the printing protein catalog and lot number 340, the nitrocellulose lot 342 used in the microarray. The glass slide etching batch 338 refers in turn to the glass slide lot 344.

Many alterations and modifications may be made by those having ordinary skill in the art without departing from the spirit and scope of the embodiments. Therefore, it must be understood that the illustrated embodiment has been set forth only for the purposes of example and that it should not be taken as limiting the embodiments as defined by the following embodiments and its various embodiments.

Therefore, it must be understood that the illustrated embodiment has been set forth only for the purposes of example and that it should not be taken as limiting the embodiments as defined by the following claims. For example, notwithstanding the fact that the elements of a claim are set forth below in a certain combination, it must be expressly understood that the embodiments includes other combinations of fewer, more or different elements, which are disclosed in above even when not initially claimed in such combinations. A teaching that two elements are combined in a claimed combination is further to be understood as also allowing for a claimed combination in which the two elements are not combined with each other but may be used alone or combined in other combinations. The excision of any disclosed element of the embodiments is explicitly contemplated as within the scope of the embodiments.

The words used in this specification to describe the various embodiments are to be understood not only in the sense of their commonly defined meanings, but to include by special definition in this specification structure, material or acts beyond the scope of the commonly defined meanings. Thus if an element can be understood in the context of this specification as including more than one meaning, then its use in a claim must be understood as being generic to all possible meanings supported by the specification and by the word itself.

The definitions of the words or elements of the following claims are, therefore, defined in this specification to include not only the combination of elements which are literally set forth, but all equivalent structure, material or acts for performing substantially the same function in substantially the same way to obtain substantially the same result. In this sense it is therefore contemplated that an equivalent substitution of two or more elements may be made for any one of the elements in the claims below or that a single element may be substituted for two or more elements in a claim. Although elements may be described above as acting in certain combinations and even initially claimed as such, it is to be expressly understood that one or more elements from a claimed combination can in some cases be excised from the combination and that the claimed combination may be directed to a sub-combination or variation of a sub-combination.

Insubstantial changes from the claimed subject matter as viewed by a person with ordinary skill in the art, now known or later devised, are expressly contemplated as being equivalently within the scope of the claims. Therefore, obvious substitutions now or later known to one with ordinary skill in the art are defined to be within the scope of the defined elements.

## Claims

1. An automated system communicated to a remote server for diagnostically field testing a sample taken from a subject using an automated portable handheld instrument to determine the presence of viral antigens and/or antibodies thereto, comprising:

   a microfluidic circuit defined in a rotatable disk;
   a bio-detector operationally positioned in the microfluidic circuit, wherein the microfluidic circuit is configured for performing a bioassay using the bio-detector to generate an electrical signal indicative of a bioassay measurement;
   one or more lasers;
   one or more positionable valves in the microfluidic circuit; and
   a backbone unit for rotating the disk according to a predetermined protocol to perform the bioassay, for controlling and powering the one or more lasers to selectively open one or more positionable valves in the microfluidic disk, for operating the bio-detector to generate the electrical signal indicative of the bioassay measurement; for communicating the bioassay measurement to the remote server, and for associating the performed bioassay and its corresponding bioassay measurement to the subject.

2. The system of claim 1, wherein the bio-detector comprises a microarray.

3. The system of claim 2, wherein the bioassay is a serology test provided by the microarray.

4. The system of claim 3, wherein the serology test includes testing for IgG and/or IgM, and/or wherein the serology test is a respiratory antibody and/or antigen test.

5. The system of any of the claims 3-4, wherein the serology test tests for Covid-19.

**6.** The system of any of the claims 2-5, wherein the microfluidic disk has a center and comprises:

a sample inlet;
a blood-plasma separation chamber communicated with the sample inlet and positioned on the disk radially farther from the center of the disk than the sample inlet;
a mixing chamber communicated to the blood-plasma separation chamber through a corresponding selectively openable valve and positioned on the disk radially farther from the center of the disk than the blood-plasma separation chamber;
a first wash chamber communicated to the mixing chamber through a corresponding selectively openable valve and positioned on the disk radially closer to the center of the disk than the mixing chamber;
a secondary antibody chamber communicated to the mixing chamber through a corresponding selectively openable valve and positioned on the disk radially closer to the center of the disk than the mixing chamber;
a second wash chamber communicated to the mixing chamber through a corresponding selectively openable valve and positioned on the disk radially closer to the center of the disk than the mixing chamber;
a microarray chamber communicated to the mixing chamber, the microarray being disposed in the microarray chamber; and the microarray chamber positioned on the disk radially farther from the center of the disk than the mixing chamber; and
a waste chamber communicated to the microarray chamber by a siphon and by a corresponding selectively openable spin-dry valve and positioned on the disk radially farther from the center of the disk than the microarray chamber.

**7.** The system of any of the claims 2-6, wherein the signal indicative of the bioassay measurement is a digital image of microarray spots which have been fluoroscopically activated by the sample in the performance of the bioassay, wherein the remote server is a Cloud server,
wherein the backbone unit includes network circuitry that communicates the digital image to the Cloud server and a corresponding schema file associating the subject to the performed bioassay and its corresponding bioassay measurement;
wherein the Cloud server, operating in an automated and modular protocol, aligns the microarray spots of the digital image, detects each of the aligned spots of the microarray and analyzes each of the spots of the digital image to assign a scalar value to each microarray spot to produce a processed microarray measurement set of data;
wherein the Cloud server, operating in an automated protocol, analyzes the processed microarray measurement set of data to produce a diagnosis of the bio-measurement; and
wherein the Cloud server, operating in an automated protocol, reports the results to the subject as determined by the schema file.

**8.** The system of claim 7, wherein the Cloud server comprises a cloud-based module for automatically determining under automated control whether corresponding Z-scores of the communicated data output of positive and/or negative indications are indicative of Covid-19 rather than Z-scores of the plurality of viral infections sharing at least some of the Covid-19 antigens and/or antibodies.

**9.** The system of claim 7 or 8, wherein the Cloud server comprises means for identifying positive and/or negative indications of the digital image of microarray spots for a plurality of acute respiratory infections selected from the group including SARS-CoV-2, SARS-CoV, MERS-CoV, common cold coronaviruses (HKU1 , OC43, NL63, 229E), and multiple subtypes of influenza, adenovirus, metapneumovirus, parainfluenza, and/or respiratory syncytial virus.

**10.** The system of any of the claims 7-9, wherein the Cloud server comprises a cloud-based module for automatically evaluating antigens to discriminate output data of a positive group of antigens from a negative group of antigens across a range of assay cutoff values using receiver-operating-characteristic (ROC) curves for which an area-under curve (AUC) is measured to determine high performing antigens to diagnose Covid-19.

**11.** The system of any of the claims 7-10, wherein the Cloud server comprises a cloud based module for automatically determining under automated control an optimal sensitivity and specificity for Covid-19 from a combination of a plurality of high performing antigens based on a corresponding Youden Index calculated for the combination of plurality of high-performing antigens.

**12.** The system of claim 5, comprising a fluoroscopic microarray reader;
wherein the remote server is a Cloud server, and wherein the microfluidic circuit is configured for performing a bioassay using the bio-detector to generate a digital image indicative of a bioassay measurement;

wherein the backbone unit is further configured for operating the fluoroscopic microarray reader to generate the digital image indicative of the bioassay measurement, and for communicating the digital image to the cloud-based server;

wherein the microfluidic disk has a center and comprises:

a sample inlet;

a blood-plasma separation chamber communicated with the sample inlet and positioned on the disk radially farther from the center of the disk than the sample inlet;

a mixing chamber communicated to the blood-plasma separation chamber through a corresponding selectively openable valve and positioned on the disk radially farther from the center of the disk than the blood-plasma separation chamber;

a first wash chamber communicated to the mixing chamber through a corresponding selectively openable valve and positioned on the disk radially closer to the center of the disk than the mixing chamber;

a secondary antibody chamber communicated to the mixing chamber through a corresponding selectively openable valve and positioned on the disk radially closer to the center of the disk than the mixing chamber;

a second wash chamber communicated to the mixing chamber through a corresponding selectively openable valve and positioned on the disk radially closer to the center of the disk than the mixing chamber;

a microarray chamber communicated to the mixing chamber, the microarray being disposed in the microarray chamber; and the microarray chamber positioned on the disk radially farther from the center of the disk than the mixing chamber; and

a waste chamber communicated to the microarray chamber by a siphon and by a corresponding selectively openable spin-dry valve and positioned on the disk radially farther from the center of the disk than the microarray chamber.

wherein the backbone unit includes network circuitry that communicates the digital image to the Cloud server and a corresponding schema file associating the subject to the performed bioassay and its corresponding bioassay measurement;

wherein the Cloud server, operating in an automated and modular protocol, aligns the microarray spots of the digital image, detects each of the aligned spots of the microarray and analyzes each of the spots of the digital image to assign a scalar value to each microarray spot to produce a processed microarray measurement set of data;

wherein the Cloud server, operating in an automated protocol, analyzes the processed microarray measurement set of data to produce a diagnosis of the bio-measurement; and

wherein the Cloud server, operating in an automated protocol, reports the results to the subject as determined by the schema file.

13. The system of claim 12, wherein the Cloud server comprises a cloud-based module for automatically determining under automated control whether corresponding Z-scores of the communicated data output of positive and/or negative indications are indicative of Covid-19 rather than Z-scores of the plurality of viral infections sharing at least some of the Covid-19 antigens and/or antibodies

14. The system of claim 12 or 13, wherein the Cloud server comprises means for identifying positive and/or negative indications of the digital image of microarray spots for a plurality of acute respiratory infections selected from the group including SARS-CoV-2, SARS-CoV, MERS-CoV, common cold coronaviruses (HKU1 , OC43, NL63, 229E), and multiple subtypes of influenza, adenovirus, metapneumovirus, parainfluenza, and/or respiratory syncytial virus.

15. The system of any of the claims 12-14, wherein the Cloud server comprises a cloud-based module for automatically evaluating antigens to discriminate output data of a positive group of antigens from a negative group antigens across a range of assay cutoff values using receiver-operating-characteristic (ROC) curves for which an area-under curve (AUC) is measured to determine high performing antigens to diagnose Covid-19; and/or

wherein the Cloud server comprises a cloud based module for automatically determining under automated control an optimal sensitivity and specificity for Covid-19 from a combination of a plurality of high performing antigens based on a corresponding Youden Index calculated for the combination of plurality of high-performing antigens.

FIG. 1

FIG. 2

FIG. 3

FIG. 4

FIG. 5

EP 3 836 152 A1

OEM
Barcode
Reader

Power
Connector    50W
            mn

24

34

Power
Supply

43

Raspberry Pi 4

5V 0.5-1A
CPU

5V 2A USB

Power
Connection

uProcessor
ARM Based
With Linux OS

Software Image
Signal Processing

I/O

Wireless Module
Without Antenna

22

Power
Switch

RGB LED

71

Power
Management

I/O

Clk

OSC

35

LPDDR2
RAM
Memory

EMMC
Flash
Memory

HDMI Micro

Display

USB 3.1

I/O          PWM

12

no EEPROM or FRAM or
Flash for Application Data
Storage. All on SD-Card

37        39

2 x Speaker
Stereo

93

IMU 6dof
Bosch Sensor Tec

USB

USB

Micro
SD Card

Audio
Amp

I/O

95

Microphone
Digikey
Adafruit

USB

USB Hub
7 Port
Geekworm
7 Port

61

65

0.1-0.3W

Headphone
Speaker

Fan 5V

97

GNSS Module
With Antenna
Built In
uBlox Chip

USB

63

55

99

Mouse
Keyboard

USB

42

89

OEM Barcode
Reader Diamond

USB

91

Cloud

134

*FIG. 5*
*CONTINUED*

*FIG. 6*

268

268

268

268

269

D8 D6

D10

D4

D3

D2

D5

D1

D7 D9

*FIG. 7*

68

92

94

104 110

102

108

100

106

114

94

72

93

74

96

92

98

112

193 — Sample Inserted

195 — Open Laser Valve 96

197 — 40 Cycles Reciprocation

199 — Open Laser Valve 106

201 — 20 Cycles Reciprocation Wash #1

203 — Open Laser Valve 108

205 — 20 Cycles Reciprocation Secondary Antibody

207 — Open Laser Valve 110

209 — 20 Cycles Reciprocation Wash #2

211 — Open Laser Valve 112

213 — Spin Dry

215 — Measure Microarray

*FIG. 8*

Scan QR ⟋216

Scan Disc → Spin Motor → Capture Image → Send Image

222 224

218 220

*FIG. 9*

216

QA Test Harness

Operator Interface (HMI) 218

222

CLI (Python)

JSON          JSON

220

Device Control

224

Cloud Send/Resp

Hardware Control

226

DataBase

Backend

230          228

*FIG. 10*

Scan QR Code          Run Test

WiFi Connection Failed

For Research Use ONLY! Not for use in diagnostic procedures.

## FIG. 11

SN:       20200729-am
HW:       E20.1
SW:       1.0
IP:       No IP Address

Normal

Scan the QR code on your phone to download WiFi Configuration App.

Press Auto WiFi Config and hold the App generated QR code in front of the Device QR scanner.

Auto WiFi Config

## FIG. 12

FIG. 13

236

Alignment

238

Spot
Detection

240

Spot
Analysis

*FIG. 14*

242

Image

JSON
Schema

244

236

Alignment

246

Cropped
Image

JSON

244

238

Spot Detection

*FIG. 15*

246

Cropped
Image

JSON

244

240

Spot Analysis

JSON

244

FIG. 16A

FIG. 16B

*FIG. 17*

FIG. 18A

FIG. 18B

FIG. 18C

*FIG. 18D*

| | 1 | 2 | 3 | 4 | 5 | 6 |
|---|---|---|---|---|---|---|
| 1 | Fiducial | BUFFER | BUFFER | BUFFER | BUFFER | BUFFER |
| 2 | PBSTwash | PBSTwash | a.HuIgG_0.3 | a.HuIgG_0.1 | a.HuIgG_0.03 | HuIgG_0.3 |
| 3 | PBSTwash | HuIgM_0.3 | HuIgM_0.14 | HuIgM_0.06 | HuIgM_0.028 | HuIgM_0.0128 |
| 4 | PBSTwash | SGC-SPIKE19200701 | PBSTwash | SARS-CoV2.RBD.mFc | PBSTwash | SARS-CoV2.NP |
| 5 | SGC-SPIKE19200701 | PBSTwash | HuIgG_0.3 | HuIgG_0.14 | HuIgG_0.06 | HuIgG_0.028 |
| 6 | HuIgM_0.06 | HuIgM_0.028 | HuIgM_0.0128 | HuIgM_0.006 | HuIgM_0.0026 | HuIgM_0.001 |
| 7 | SARS-CoV2.RBD.mFc | PBSTwash | SARS-CoV2.NP | PBSTwash | SGC-SPIKE19200701 | PBSTwash |
| 8 | HuIgG_0.14 | HuIgG_0.06 | HuIgG_0.028 | HuIgG_0.0128 | HuIgG_0.006 | HuIgG_0.0026 |
| 9 | HuIgM_0.006 | HuIgM_0.0026 | HuIgM_0.001 | PBSTwash | HuIgG_0.3 | HuIgG_0.14 |
| 10 | HuIgM_0.3 | HuIgM_0.14 | HuIgM_0.06 | HuIgM_0.028 | HuIgM_0.0128 | HuIgM_0.006 |
| 11 | HuIgG_0.0128 | HuIgG_0.006 | HuIgG_0.0026 | HuIgG_0.001 | PBSTwash | HuIgM_0.3 |
| 12 | PBSTwash | SARS-CoV2.RBD.mFc | PBSTwash | SARS-CoV2.NP | PBSTwash | SGC-SPIKE19200701 |
| 13 | Fiducial | PBSTwash | PBSTwash | a.HuIgM_0.03 | a.HuIgM_0.1 | a.HuIgM_0.3 |

# FIG. 19

| 7 | 8 | 9 | 10 | 11 | 12 | 13 |
|---|---|---|---|---|---|---|
| BUFFER | BUFFER | BUFFER | BUFFER | BUFFER | BUFFER | Fiducial |
| HuIgG_0.14 | HuIgG_0.06 | HuIgG_0.028 | HuIgG_0.0128 | HuIgG_0.006 | HuIgG_0.0026 | HuIgG_0.001 |
| HuIgM_0.006 | HuIgG_0.0026 | HuIgM_0.001 | PBSTwash | SARS-CoV2.RBD.mFc | PBSTwash | SARS-CoV2.NP |
| PBSTwash | SGC-SPIKE19200701 | PBSTwash | SARS-CoV2.RBD.mFc | PBSTwash | SARS-CoV2.NP | PBSTwash |
| HuIgG_0.0128 | HuIgG_0.006 | HuIgG_0.0026 | HuIgG_0.001 | PBSTwash | HuIgM_0.3 | HuIgM_0.14 |
| PBSTwash | SARS-CoV2.RBD.mFc | PBSTwash | SARS-CoV2.NP | PBSTwash | SGC-SPIKE19200701 | PBSTwash |
| SARS-CoV2.RBD.mFc | PBSTwash | SARS-CoV2.NP | PBSTwash | SGC-SPIKE19200701 | PBSTwash | HuIgG_0.3 |
| HuIgG_0.001 | PBSTwash | HuIgM_0.3 | HuIgM_0.14 | HuIgM_0.06 | HuIgM_0.028 | HuIgM_0.0128 |
| HuIgG_0.06 | HuIgG_0.028 | HuIgG_0.0128 | HuIgG_0.006 | HuIgG_0.0026 | HuIgG_0.001 | PBSTwash |
| HuIgM_0.0026 | HuIgM_0.001 | PBSTwash | HuIgG_0.3 | HuIgG_0.14 | HuIgG_0.06 | HuIgG_0.028 |
| HuIgM_0.14 | HuIgM_0.06 | HuIgM_0.028 | HuIgM_0.0128 | HuIgM_0.006 | HuIgM_0.0026 | HuIgM_0.001 |
| PBSTwash | SARS-CoV2.RBD.mFc | PBSTwash | SARS-CoV2.NP | PBSTwash | SGC-SPIKE19200701 | PBSTwash |
| BLANK | BLANK | BLANK | BLANK | BLANK | BLANK | BLANK |

FIG. 19
CONTINUED

FIG. 20

FIG. 21A

//////////////////////////DAY 2////////////////////////////

**408**

Patient Goes To Test Location And Scans QR Code

**426**

Patient Blood Is Drawn And Placed into Disc

**410** **418** **420** **430**

Instrument Prompt: "Scan QR Code"

Instrument Prompt: "Scan Disc QR Code"

Disc Metadata Is Downloaded From Cloud And Loaded Onto Device. Disc OK To Use?

YES

TO 432

Disc Is Loaded Into Device

NO

Determines If QR Code Is Valid. Sends Disc Data To Test Location And Prompts Instrument To Go Ahead With Test

**412**

Cloud Sends Disc Metadata To Device:
• Status Of Disc Batch
• JSON Spin Recipe
• Tif With Images Of Arrays During QC

**422**

**414** **416**

Operator Removes Disc From Packaging And Ensures Temp/ Humidity/UV Indicators Show Disc Is Good

Operator Scans Disc QR Code

Operator Rejects Disc And Takes Another One

Operator Takes Blood From Patient And Loads Into Disc

**424** **428**

## FIG. 21B

**432**

**436**

FROM
420

**Instrument Prompt: "Start Test"**

**Pre-Test diagnostic Data Is Gathered**

Optical System Checked By Taking Images Of Microarray A And B:
- Verify 3 Fiducials Are Visible
- Verify Fiducial Intensity Within 20% Of Original Images
- Verify Fiducials Are In Focus

**Watchdog Microcontroller Outputs Diagnostic Data From Camera, LEDs, Motor, Lasers**

TO
442

**438**

**440**

**434**

**Operator Pushes "Start Test" Button**

*FIG. 21C*

442

Instrument Runs Spin Recipe On Device And Takes
Images Of Microarrays At End Of Test

FROM
436

Watchdog Microcontroller
Monitors Device And Throws
Error To Stop Test If Needed

444

Tif Uploaded To Cloud Containing
The Following information:
• Microarray A And B Images Taken
   Before Test (Background Data)
• Microarray A And B Images Taken
   After Test
• Diagnostic Data Taken From
   Before And During Test

Image Processing
Alogorithm
Generate JSON
With Spot Name,
Spot Location, And
Fluorescent
Intensity

TO
450

446

448

FIG. 21D

FIG. 21E

FIG. 22

FIG. 22A

FIG. 22B

FIG. 22C

FIG. 23

TABLE 1

| name | value IgG | ref. IgG | z.score IgG | value IgM | ref. IgM | z.score IgM |
|---|---|---|---|---|---|---|
| SARS.CoV.2.NP | 14551.00 | 1374.91 | 11.19 | 1729.80 | 1420.89 | 0.17 |
| SARS.CoV.2.Pl.pro | 403.50 | 685.28 | -0.40 | 180.72 | 153.55 | 0.12 |
| SARS.CoV.2.S1 | 2553.60 | 1695.89 | 0.61 | 1379.55 | 625.83 | 2.68 |
| SARS.CoV.2.S1.HisTag | 3673.05 | 250.83 | 15.86 | 1588.15 | 67.92 | 11.26 |
| SARS.CoV.2.S1.mFcTag | 7440.25 | 545.47 | 13.16 | 5666.20 | 659.51 | 9.57 |
| SARS.CoV.2.S1.RBD | 7467.90 | 570.75 | 12.01 | 6846.95 | 1273.64 | 10.58 |
| SARS.CoV.2.S1+S2 | 7518.00 | 2233.85 | 2.88 | 3435.40 | 989.59 | 5.88 |
| SARS.CoV.2.S2 | 2452.55 | 1278.24 | 0.97 | 1178.90 | 583.27 | 3.11 |
| SARS.CoV.2.Spike.RBD.Bac | 2254.20 | 888.46 | 3.34 | 2099.50 | 520.37 | 4.72 |
| SARS.CoV.2.Spike.RBD.His.HEK | 2609.60 | 278.67 | 9.42 | 2110.35 | 115.50 | 14.17 |
| SARS.CoV.2.Spike.RBD.rFc | 4981.15 | 839.19 | 9.79 | 2974.90 | 618.89 | 6.03 |
| SARS.CoV_NP | 14529.50 | 2759.59 | 10.41 | 3772.35 | 2415.02 | 0.66 |
| SARS.CoV_PLpro | 1069.70 | 583.84 | 1.49 | 292.55 | 423.26 | -0.51 |
| SARS.CoV_S1.HisTag | 679.55 | 1418.36 | -0.85 | 276.60 | 856.47 | -1.67 |
| SARS.CoV_S1.RBD.HisTag | 502.40 | 873.32 | -0.87 | 227.70 | 448.94 | -0.94 |
| SARS.CoV_S1.RBD.rFcTag | 853.15 | 1669.10 | -1.39 | 637.10 | 925.24 | -0.59 |
| MERS.CoV_NP | 501.15 | 1878.35 | -0.55 | 1208.60 | 1569.42 | -0.13 |
| MERS.CoV_S1.AA1.725.His.HEK | 137.65 | 303.19 | -0.85 | 21.85 | 111.41 | -0.61 |
| MERS.CoV_S1.RBD.367.606.rFcTag | 1141.75 | 3405.61 | -1.78 | 775.00 | 1034.58 | -0.47 |
| MERS.CoV_S1.RBD.383.502.mFcTag | 373.45 | 1265.92 | -0.99 | 1247.55 | 2310.08 | -0.82 |
| MERS.CoV_S2 | 4554.95 | 2780.55 | 0.83 | 455.75 | 946.69 | -0.77 |
| DcCoV.HKU23.NP | 917.55 | 2571.47 | -0.97 | 352.05 | 588.45 | -0.51 |
| hCoV.229E.S1 | 3155.40 | 5439.22 | -1.01 | 167.12 | 372.36 | -0.82 |
| hCoV.229E.S1_S2 | 7544.50 | 10036.24 | -1.03 | 615.45 | 1927.28 | -0.53 |
| hCoV.HKU1.HE | 3360.50 | 6264.33 | -0.88 | 1380.10 | 4284.79 | -1.02 |
| hCoV.HKU1.S1_AA1.760 | 1648.75 | 2920.27 | -0.78 | 53.15 | 180.58 | -1.23 |
| hCoV.HKU1.S1_AA13.756 | 998.35 | 3012.07 | -0.94 | 251.50 | 422.75 | -1.05 |
| hCoV.HKU1.S1_S2 | 6798.20 | 4890.55 | 0.95 | 538.55 | 1013.67 | -1.37 |
| hCoV.NL63.S1 | 1281.60 | 1659.04 | -0.52 | 124.70 | 253.78 | -0.85 |
| hCoV.NL63.S1_S2 | 2030.60 | 3302.70 | -1.17 | 394.60 | 1026.94 | -0.77 |
| hCoV.OC43.HE | 1263.10 | 2992.68 | -1.11 | 153.25 | 447.21 | -1.01 |
| hCoV.OC43.S1 | 212.90 | 383.06 | -0.74 | 67.05 | 223.34 | -1.17 |
| hCoV.OC43.S1_S2 | 12497.90 | 7958.39 | 2.01 | 841.28 | 1169.18 | -0.61 |
| Flu.B_Mal.HA1 | 7884.25 | 8558.08 | -0.23 | 184.90 | 438.99 | -0.45 |
| Flu.B_Mal.HA1+HA2 | 11362.85 | 11918.07 | -0.24 | 446.90 | 515.63 | -0.12 |
| Flu.B_Phu.HA1 | 7333.90 | 6356.85 | 0.32 | 175.35 | 332.97 | -0.52 |
| Flu.B_Phu.HA1+HA2 | 10142.05 | 11923.46 | -0.67 | 858.40 | 1997.51 | -0.55 |
| Flu.H1N1.HA1 | 1731.10 | 4421.82 | -1.03 | 252.75 | 489.13 | -1.12 |
| Flu.H1N1.HA1+HA2 | 10957.75 | 10597.92 | 0.10 | 1187.55 | 576.35 | 0.86 |
| Flu.H3N2.HA1 | 12223.65 | 8603.17 | 1.17 | 260.70 | 336.37 | -0.24 |
| Flu.H3N2.HA1+HA2 | 13491.35 | 11237.43 | 0.78 | 696.30 | 1184.50 | -0.56 |
| Flu.H5N1.HA1 | 1645.55 | 3725.37 | -0.98 | 931.65 | 1504.46 | -0.82 |
| Flu.H5N1.HA1+HA2 | 7285.50 | 9349.40 | -0.63 | 1855.95 | 1730.49 | 0.15 |
| Flu.H7N9.HA1 | 654.45 | 1138.46 | -0.59 | 82.65 | 117.95 | -1.23 |
| Flu.H7N9.HA1+HA2 | 838.35 | 1501.44 | -0.59 | 16.00 | 103.03 | -2.19 |
| hAdV3.Fiber | 3108.70 | 5882.67 | -0.62 | 820.25 | 857.55 | -0.10 |
| hAdV3.Penton | 2758.50 | 3406.34 | -0.35 | 796.30 | 624.49 | 0.13 |
| hAdV4.Fiber | 4197.65 | 3940.21 | 0.08 | 519.55 | 640.59 | -0.33 |
| hAdV4.Penton | 1466.70 | 2241.25 | -0.44 | 490.85 | 640.63 | -0.45 |
| hMPV.A_G.52N.228N | 603.80 | 1741.45 | -1.59 | 496.10 | 816.97 | -0.39 |
| hMPV.B_F.280D.490G | 275.70 | 746.49 | -0.91 | 309.00 | 452.87 | -0.47 |
| hMPV.B_G.52D.238S | 560.40 | 1016.13 | -0.44 | 197.10 | 474.71 | -0.75 |
| hPIV.1.1203_F | 5352.15 | 7393.48 | -0.83 | 786.92 | 1315.02 | -0.93 |
| hPIV.1.1203_H | 4208.50 | 7259.00 | -1.50 | 1081.15 | 2833.11 | -1.15 |
| hPIV.3.2010_H | 6143.55 | 7617.24 | -0.76 | 840.20 | 1376.41 | -0.90 |
| hPIV.4.b.2016_H | 1839.00 | 4052.51 | -1.16 | 811.40 | 1287.62 | -0.90 |
| RSV.A.F | 8134.12 | 9708.27 | -1.86 | 473.80 | 1150.34 | -1.17 |
| RSV.A.G | 4016.20 | 9260.15 | -1.93 | 855.55 | 629.59 | 0.57 |
| RSV.B.F | 10774.30 | 11656.42 | -0.49 | 1036.20 | 1529.28 | -0.39 |
| RSV.B.G | 8945.12 | 11369.46 | -0.87 | 1356.35 | 762.25 | 0.89 |

# FIG. 24

TABLE 2

| N | Antigen Combination | IgG AUC | IgG Spec | IgG Sens |
|---|---|---|---|---|
| 1 | SARS-CoV-2_S1+S2 | 0.975 | 0.987 | 0.889 |
| 1 | SARS-CoV-2_NP | 0.975 | 0.961 | 0.889 |
| 1 | SARS-CoV-2_S2 | 0.951 | 0.921 | 0.833 |
| 1 | SARS-CoV_NP | 0.957 | 0.974 | 0.833 |
| 1 | SARS-CoV-2_S1 (mFcTag) | 0.88 | 0.987 | 0.667 |
| 1 | MERS-CoV_S2 | 0.873 | 0.763 | 0.889 |
| 1 | SARS-CoV-2_S1-RBD | 0.849 | 0.947 | 0.833 |
| 2 | SARS-CoV-2_NP ; MERS-CoV_S2 | 0.988 | 0.934 | 1 |
| 2 | SARS-CoV-2_S1+S2 ; SARS-CoV-2_NP | 0.988 | 0.963 | 0.947 |
| **2** | **SARS-CoV-2_S1+S2 ; SARS-CoV_NP** | 0.975 | 0.974 | 0.889 |
| **2** | **SARS-CoV-2_S2 ; SARS-CoV_NP** | **0.994** | **1** | **0.944** |
| 3 | SARS-CoV-2_NP ; SARS-CoV-2_S2 ; SARS-CoV_NP | 0.988 | 1 | 0.944 |
| 3 | SARS-CoV-2_S1+S2 ; SARS-CoV-2_NP ; SARS-CoV_NP | 0.981 | 1 | 0.889 |
| 3 | SARS-CoV-2_S1+S2 ; SARS-CoV-2_S2 ; SARS-CoV_NP | 0.975 | 1 | 0.889 |
| **3** | **SARS-CoV-2_S1+S2 ; SARS-CoV_NP ; SARS-CoV-2_S1, (mFcTag)** | **0.969** | 0.961 | 0.889 |
| 3 | SARS-CoV-2_S2 ; SARS-CoV_NP ; MERS-CoV_S2 | 0.988 | 1 | 0.944 |
| **3** | **SARS-CoV-2_S2 ; SARS-CoV_NP ; SARS-CoV-2_S1, (mFcTag)** | **0.994** | **1** | **0.944** |
| 4 | SARS-CoV-2_S1+S2 ; SARS-CoV-2_NP ; SARS-CoV-2_S2 ; SARS-CoV_NP | 0.981 | 1 | 0.944 |
| 4 | SARS-CoV-2_S1+S2 ; SARS-CoV-2_NP ; SARS-CoV_NP ; SARS-CoV-2_S1-RBD | 0.975 | 1 | 0.833 |
| 4 | SARS-CoV-2_S1+S2 ; SARS-CoV-2_S2 ; SARS-CoV_NP ; SARS-CoV-2_S1, (mFcTag) | 0.981 | 0.987 | 0.944 |
| 4 | SARS-CoV-2_S1+S2 ; SARS-CoV_NP ; MERS-CoV_S2 ; SARS-CoV-2_S1-RBD | 0.975 | 1 | 0.944 |
| 4 | SARS-CoV-2_S2 ; SARS-CoV_NP ; SARS-CoV-2_S1, (mFcTag) ; SARS-CoV-2_S1-RBD | 0.988 | 1 | 0.944 |

# FIG. 25

Europäisches
Patentamt

European
Patent Office

Office européen
des brevets

# EUROPEAN SEARCH REPORT

Application Number

EP 20 21 1391

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/201900 A1 (SHACHAR JOSH YEHOSHUA [US] ET AL) 4 July 2019 (2019-07-04) | 1-4,6 | INV. G16H10/40 |
| Y | * par. [0017]-[0044], [0082], [0133], [0150], [0151], [0168], figures 11 and 12. * | 5,7-15 | G16H50/80 G01N33/543 B01L3/00 |
| | ----- | | |
| Y,P | Khan Saahir ET AL: "Analysis of Serologic Cross-Reactivity Between Common Human Coronaviruses and SARS-CoV-2 Using Coronavirus Antigen Microarray", bioRxiv : the preprint server for biology, 25 March 2020 (2020-03-25), pages 1-10, XP055800048, DOI: 10.1101/2020.03.24.006544 Retrieved from the Internet: URL:https://www.biorxiv.org/content/10.1101/2020.03.24.006544v1.full.pdf [retrieved on 2021-04-29] * abstract; page 4, 2nd and 3rd paragraph; page 5, 2nd par. * | 5,7-15 | |
| | ----- | | TECHNICAL FIELDS SEARCHED (IPC) |
| Y,P | Assis Rafael R ET AL: "Analysis of SARS-CoV-2 Antibodies in COVID-19 Convalescent Plasma using a Coronavirus Antigen Microarray", bioRxiv : the preprint server for biology, 17 April 2020 (2020-04-17), pages 1-21, XP055800069, DOI: 10.1101/2020.04.15.043364 Retrieved from the Internet: URL:https://www.biorxiv.org/content/10.1101/2020.04.15.043364v1.full.pdf [retrieved on 2021-04-29] * abstract; page 4, 2nd paragraph - page 6, 2nd par., the figures. * | 5,7-15 | G16H G01N B01L |
| | ----- | | |
| | -/-- | | |

The present search report has been drawn up for all claims

| Place of search | Date of completion of the search | Examiner |
|---|---|---|
| Munich | 30 April 2021 | Lindberg, Pia |

EPO FORM 1503 03.82 (P04C01)

page 1 of 2

Europäisches
Patentamt

European
Patent Office

Office européen
des brevets

# EUROPEAN SEARCH REPORT

**Application Number**

EP 20 21 1391

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2006/047697 A1 (CONWAY TYRELL [US] ET AL) 2 March 2006 (2006-03-02) <br> * abstract; <br> paragraphs [0067]-[0073]; <br> the claims * | 1-15 | |
| A | TRIVEDI SUVANG U. ET AL: "Development and Evaluation of a Multiplexed Immunoassay for Simultaneous Detection of Serum IgG Antibodies to Six Human Coronaviruses", SCIENTIFIC REPORTS, vol. 9, no. 1, 1 December 2019 (2019-12-01), page 1390, XP055799533, DOI: 10.1038/s41598-018-37747-5 Retrieved from the Internet: URL:https://www.ncbi.nlm.nih.gov/pmc/artic les/PMC6362100/pdf/41598_2018_Article_3774 7.pdf> <br> * the whole document * | 1-15 | |
| A,P | Perkmann Thomas ET AL: "Side by side comparison of three fully automated SARS-CoV-2 antibody assays with a focus on specificity", medRxiv, 9 June 2020 (2020-06-09), XP055799579, DOI: 10.1101/2020.06.04.20117911 Retrieved from the Internet: URL:https://www.medrxiv.org/content/10.110 1/2020.06.04.20117911v3.full.pdf [retrieved on 2021-04-28] <br> * the whole document * | 1-15 | TECHNICAL FIELDS SEARCHED (IPC) |

The present search report has been drawn up for all claims

| Place of search | Date of completion of the search | Examiner |
|---|---|---|
| Munich | 30 April 2021 | Lindberg, Pia |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 21 1391

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2021

| Patent document cited in search report | | Publication date | Patent family member(s) | Publication date |
|---|---|---|---|---|
| US 2019201900 | A1 | 04-07-2019 | NONE | |
| US 2006047697 | A1 | 02-03-2006 | NONE | |

EPO FORM P0459

For more details about this annex : see Official Journal of the European Patent Office, No. 12/82